# EUROPEAN PATENT APPLICATION

(11) **EP 4 711 462 A1**
(43) Date of publication of application: **18.03.2026**
(21) Application number: 24807169.8
(22) Date of filing: 10.05.2024
(51) Int. Cl.: C12N 15/13, A61K 9/19, A61K 39/395, A61P 31/04, C07K 16/12, C12N 1/15, C12N 1/19, C12N 1/21, C12N 5/10, C12N 15/63, C12N 15/85, C12P 21/08

(54) **ANTIBODY THAT BINDS TO CLOSTRIDIOIDES DIFFICILE CELLS**

(30) Priority: 12.05.2023 WO PCT/JP2023/018019; 10.08.2023 JP 2023131537
(71) Applicant: The University of Tokyo, Bunkyo-ku, Tokyo 113-8654 (JP)
(72) Inventor: SHINKURA Reiko, Tokyo 113-8654 (JP); TAMANO Ryotaro, Tokyo 113-8654 (JP); TAKAHASHI Keishyuu, Tokyo 113-8654 (JP)
(74) Representative: Grünecker Patent- und Rechtsanwälte PartG mbB
(86) International application number: PCT/JP2024/017523
(87) International publication number: WO 2024/237217

(57) **Abstract**

As a result of intensive studies by the inventors of the present disclosure, the inventors have found an antibody that binds to enteric bacteria, particularly bacterial cells of C. difficile and bacterial cells of other enteric bacteria, and inhibits their growth. The present disclosure has an effect of providing an antibody that binds to enteric bacteria, particularly bacterial cells of C. difficile and bacterial cells of other enteric bacteria, and providing an antigen-binding fragment of the antibody and uses of the antibody and the antigen-binding fragment.

## Description

### Technical Field

The present disclosure relates to an antibody that binds to Clostridioides difficile cells (C. difficile cells) and has, for example, an effect of inhibiting growth of the C. difficile cells, and relates to an antigen-binding fragment of the antibody and a use of the antibody.

### Background Art

The inside of the intestinal tract is so-called the "outside of the body" for a living body, and thus is always exposed to antigens such as a very wide variety and a large number of intestinal resident bacteria, viruses, and antigens derived from foods. Such intestinal resident bacteria form an intestinal bacterial flora.

It has been clear that the intestinal bacterial flora performs various functions on a living body via a mucosal surface including intestinal epithelial cells and the like, and it has also been known that unless enteric bacteria are present, the intestinal immune system does not normally develop (Non Patent Literature 1 to 3).

If the intestinal bacterial flora changes to lose the symbiosis with the host, the intestinal immune system is excessively stimulated and its homeostasis collapses, and consequently, many diseases are induced such as inflammatory bowel disease, colorectal cancer, asthma, allergy, and obesity. From these facts, it has been known that the intestinal immune system plays an important role not only in eliminating pathogens and the like but also in maintaining the homeostasis of the entire immune system (Non Patent Literature 4 to 7).

Many attempts have been conventionally made to control the intestinal bacterial flora using an antibiotic, but there is a problem that the control may become ineffective due to a problem of antibiotic-resistant bacteria. Thus, despite the conventional attempts, there are still many patients suffering from Clostridium difficile infection and another inflammatory bowel disease associated with an intestinal bacterial flora. In addition, attempts of a relatively new approach such as fecal transplantation have been made, but the treatment effects of such attempts are unstable. As a result, in some attempts, development of a treatment method has already been stopped. Furthermore, an antibody against a toxin released from the intestinal bacterial flora has been conventionally studied for use as a therapeutic agent, and commercialized. However, such an antibody therapeutic is only against a toxin, so that a treatment method using the antibody therapeutic is only symptomatic. Therefore, development of medicine has been desired for radically treating inflammatory bowel disease associated with the intestinal bacterial flora.

C. difficile cells related to inflammatory bowel disease require attention, together with MRSA, as cells that may cause nosocomial infection. In fact, according to U.S. statistics, about 400000 to 500000 people are infected with C. difficile cells, and about 15000 to 20000 people die every year. An effective treatment is particularly required against C. difficile cells among inflammatory bowel disease-associated bacteria in that infection with C. difficile cells often occurs in patients on long-term administration of antibiotics and one occurrence of infection results in a probability of recurrence of as high as 15 to 35%. At present, a treatment with a strong antibiotic such as vancomycin is performed, but the toxins from cells remain and emergence of cells having antibiotic resistance is promoted, and therefore an alternative technique is required.

### Citation List

### Non Patent Literature

Non Patent Literature 1: Cerf-Bensussan N, and Gaboriau-Routhiau V., Nat Rev Immunol 2010; 10: 735
Non Patent Literature 2: Hooper LV, and Macpherson AJ., Nat Rev Immunol 2010; 10: 159
Non Patent Literature 3: Round JL, and Mazmanian SK, Proc Natl Acad Sci USA 2010; 107: 12204
Non Patent Literature 4: Vijay-Kumar M, et al., Science 2010; 328: 228
Non Patent Literature 5: Shulzhenko N, et al., Nat Med 2011; 17: 1585
Non Patent Literature 6: Bry L, et al., Science 1996; 273: 1380
Non Patent Literature 7: Turnbaugh PJ, et al., Nature 2006; 444: 1027

### Summary of Invention

### Technical Problem

An object of the present disclosure is to provide an antibody that binds to bacterial cells of C. difficile, an antigen-binding fragment of the antibody, and uses of the antibody and the antigen-binding fragment.

### Solution to Problem

As a result of intensive studies by the inventors of the present disclosure, the inventors have found an antibody that binds to enteric bacteria, particularly bacterial cells of C. difficile and bacterial cells of other enteric bacteria, and inhibits their growth. The inventors have also found that the antibody further binds to bacteria present in an abnormal intestinal bacterial flora (dysbiosis).

In one aspect, the present disclosure provides an antibody that binds to bacterial cells of C. difficile and inhibits growth of the bacterial cells, or provides an antigen-binding fragment of the antibody.

In one aspect, the present disclosure provides an immortalized cell capable of producing an antibody that binds to bacterial cells of C. difficile and inhibits growth of the bacterial cells.

In one aspect, the present disclosure provides a composition containing an antibody that binds to bacterial cells of C. difficile and inhibits growth of the bacterial cells, or containing an antigen-binding fragment of the antibody.

In one aspect, the present disclosure provides a method of treating a disease using an antibody that binds to bacterial cells of C. difficile and inhibits growth of the bacterial cells, or using an antigen-binding fragment of the antibody.

In one aspect, the present disclosure provides the followings.

### [Item 1]

An antibody or an antigen-binding fragment of the antibody, comprising:
a heavy chain CDR1, a heavy chain CDR2, and a heavy chain CDR3 of a heavy chain variable region represented by SEQ ID NO: 3; and
a light chain CDR1, a light chain CDR2, and a light chain CDR3 of a light chain variable region represented by SEQ ID NO: 4, or comprising:
   a heavy chain CDR1, a heavy chain CDR2, and a heavy chain CDR3 of a heavy chain variable region represented by SEQ ID NO: 13; and
   a light chain CDR1, a light chain CDR2, and a light chain CDR3 of a light chain variable region represented by SEQ ID NO: 14,
   the antibody or the antigen-binding fragment that binds to bacterial cells of Clostridioides difficile (C. difficile) and inhibits growth of Clostridioides difficile.

### [Item 2]

The antibody or the antigen-binding fragment according to the item 1, wherein
the heavy chain CDR1 is represented by SEQ ID NO: 5, the heavy chain CDR2 is represented by SEQ ID NO: 6, and the heavy chain CDR3 is represented by SEQ ID NO: 7, and
the light chain CDR1 is represented by SEQ ID NO: 8, the light chain CDR2 is represented by SEQ ID NO: 9, and the light chain CDR3 is represented by SEQ ID NO: 10.

### [Item 3]

The antibody or the antigen-binding fragment according to the item 1, wherein
the heavy chain CDR1 is represented by SEQ ID NO: 15, the heavy chain CDR2 is represented by SEQ ID NO: 16, and the heavy chain CDR3 is represented by SEQ ID NO: 17, and
the light chain CDR1 is represented by SEQ ID NO: 18, the light chain CDR2 is represented by SEQ ID NO: 19, and the light chain CDR3 is represented by SEQ ID NO: 20.

### [Item 4]

A nucleic acid encoding the antibody or the antigen-binding fragment according to the item 1.

### [Item 5]

An expression vector comprising the nucleic acid according to the item 4.

### [Item 6]

A cell comprising the nucleic acid according to the item 4.

### [Item 7]

A composition comprising the antibody or the antigen-binding fragment according to the item 1.

### [Item 8]

The composition according to the item 7, being in a lyophilized state.

### Advantageous Effects of Invention

The present disclosure has an effect of providing an antibody that binds to enteric bacteria, particularly bacterial cells of C. difficile and bacterial cells of other enteric bacteria, and providing an antigen-binding fragment of the antibody and uses of the antibody and the antigen-binding fragment.

### Brief Description of Drawings

Fig. 1 is a graph showing results of testing the binding force of SNK0004 antibody and SNK0005 antibody to C. difficile cells.
Fig. 2 is a graph showing results of testing the C. difficile growth-inhibiting ability of SNK0004 antibody, SNK0005 antibody, and the IgG1 type antibody (rW27IgG) and the negative-control IgA antibody (Control rIgA) of W27 antibody.
Fig. 3 is a graph showing results of evaluating the binding property of each bacterial species in inflammatory bowel disease (IBD) patient feces-derived bacteria that bind to SNK0004 antibody, with quantitative data by the IgA index.
Fig. 4 is a graph showing results of evaluating the binding property of each bacterial species in IBD patient feces-derived bacteria that bind to SNK0005 antibody, with quantitative data by the IgA index.
Fig. 5 is a graph showing results of testing the ratios of IBD patient feces-derived bacteria that bind to SNK0004 and SNK0005 antibodies and previously prepared RS_H000_L001, SNK0001, SNK0002, and SNK0003 antibodies (WO2023/277142).

### Description of Embodiments

### (Definitions)

In the present description, in a case where a plurality of numerical ranges are shown, a range is also meant that includes a combination of any of the lower limits of the plurality of ranges and any of the upper limits of the plurality of ranges.

In the present description, the term "antibody" is used in the broadest sense and may refer to, but is not limited to, monoclonal antibodies, polyclonal antibodies, and antibody fragments that exhibit an intended antigen binding activity. A full-length antibody comprises a heavy chain and a light chain that mainly comprise a polypeptide. The heavy chain and the light chain each comprise a site referred to as a variable region that recognizes an antigen, and the sites are generally referred to as a heavy chain variable region and a light chain variable region, respectively. Each variable region has sites referred to as CDR1 to CDR3, respectively, in order from the amino terminus, and the sites are each identified as a site that recognizes an antigen in more detail. The CDR1 to the CDR3 are also referred to more specifically, for example, as a heavy chain CDR1, a heavy chain CDR2, a heavy chain CDR3, a light chain CDR1, a light chain CDR2, and a light chain CDR3. Regions other than the CDR1 to the CDR3 of the heavy chain and the CDR1 to the CDR3 of the light chain are referred to as a heavy chain FR1 to a heavy chain FR4 and a light chain FR1 to a light chain FR4, respectively, in order from the amino terminus.

The antibody may be in the form of an antibody comprising one heavy chain and one light chain (also referred to as a single-chain antibody) other than the form comprising two heavy chains and two light chains. The antibody may also be in the form of a dimer or multimer having a joining chain (J chain) and optionally having a secretary component (SC) in addition to two heavy chains and two light chains.

The antibody may be an antibody of any class, such as IgG, IgE, IgM, IgD, IgA, or IgY, or may be an antibody of any subclass, such as IgG1, IgG2, IgG3, IgG4, IgA1, or IgA2.

The antibody may have an amino acid sequence derived from the same species or derived from different species. In the case of the amino acid sequence derived from the same species, examples of the species include a human, a mouse, a rat, a hamster, a rabbit, a goat, a donkey, a pig, a cow, a horse, a chicken, a monkey, a chimpanzee, a camel, and a llama.

The antibody having an amino acid sequence derived from different species is also not particularly limited, and examples of the antibody include antibodies derived from any two or more of a human, a mouse, a rat, a hamster, a rabbit, a goat, a sheep, a donkey, a pig, a cow, a horse, a chicken, a monkey, a chimpanzee, a camel, a llama, and the like.

In the present description, an "antigen-binding fragment" of an antibody refers to one or more fragments of the antibody that retain the ability to specifically bind to an antigen. It has been found that a fragment comprising a part of an antibody can also maintain the ability of the antibody to specifically bind to an antigen. In one aspect, an "antigen-binding fragment" of an antibody may be, but is not limited to, a Fab fragment comprising a light chain variable region (VL), a heavy chain variable region (VH), a light chain constant region (CL), and a CH1 domain that is a part of a heavy chain constant region, a F(ab')2 fragment comprising two Fab fragments linked by a disulfide bridge at a hinge region, a Fd fragment comprising VH and CH1 domains, a Fv fragment comprising VL and VH domains of a single arm of the antibody, a dAb fragment comprising a single variable domain, and an isolated complementarity-determining region (CDR).

The antibody of the present disclosure may be a CDR-grafted antibody. In one example of a CDR-grafted antibody, some or all of the sequences of the CDR region of an antibody derived from one animal species are substituted with CDR sequences of another animal species. For example, one or more CDRs of a mouse antibody are substituted with CDR sequences of a human antibody.

The heavy chain CDR1 to the heavy chain CDR3 in the heavy chain variable region and the light chain CDR1 to the light chain CDR3 in the light chain variable region of an antibody can be identified using a method well known to those skilled in the art. For example, methods well known to those skilled in the art can be used such as "Kabat definition" (Kabat et al., Ann. NY Acad, Sci. 1971, Vol. 190, pp. 382-391 and Kabat, E. A. et al., Sequences of Proteins of Immunological Interest, 5th Ed. 1991, U.S. Department of Health and Human Services, NIH Publication, pp. 91-3242), "Chothia definition" (Chothia et al., Nature, 1989, Vol. 342, pp. 877-883), and "contact definition" (MacCallum et al., J. Mol. Biol., 1996, Vol. 262, pp. 732-745). For identifying CDR sequences in an antibody, the CDR may be identified on the basis of information from public databases (for example, https://www.ncbi.nlm.nih.gov/igblast).

In the present description, the term "identity" refers to the degree of equality of two or more comparable amino acid sequences or base sequences with each other. Therefore, the higher the identity of two amino acid sequences or base sequences, the higher the identity or similarity of the sequences. The level of identity of amino acid sequences or base sequences is usually determined using FASTA, which is a tool for sequence analysis, with default parameters. Alternatively, the level can be determined using the algorithm BLAST by Karlin and Altschul (for example, Karlin S, Altschul SF. Proc. Natl Acad Sci USA. 87: 2264-2268 (1990), Karlin S, Altschul SF. Natl Acad Sci USA. 90: 5873-7 (1993), etc.). Programs called BLASTN and BLASTX based on such a BLAST algorithm have been developed (for example, Altschul SF, GishW, Miller W, Myers EW, Lipman DJ. J Mol Biol. 215: 403-10 (1990), etc.). Specific techniques for these analysis methods are known, and the NCBI website can be referred to. For example, the fact that a certain amino acid sequence A and another amino acid sequence B are identical by a specific percentage means that the amino acid sequence A and the amino acid sequence B have an identity of the percentage.

In the present description, the term "monoclonal" is a modifier indicating a characteristic of an antibody or the like obtained from a substantially homogeneous antibody population. The antibodies included in such an antibody population are identical except for a possible naturally occurring mutation that may be present in a trace amount.

In the present description, the term "conservative substitution technique" means a technique in which an amino acid residue is substituted with an amino acid residue having a similar side chain.

Examples of the conservative substitution technique include substitution between amino acid residues having a basic side chain, such as lysine, arginine, and histidine. In addition, examples of the conservative substitution technique also include substitution between amino acid residues having an acidic side chain, such as aspartic acid and glutamic acid; amino acid residues having an uncharged polar side chain, such as glycine, asparagine, glutamine, serine, threonine, tyrosine, and cysteine; amino acid residues having a nonpolar side chain, such as alanine, valine, leucine, isoleucine, proline, phenylalanine, methionine, and tryptophan; amino acid residues having a β-branched side chain, such as threonine, valine, and isoleucine; and amino acid residues having an aromatic side chain, such as tyrosine, phenylalanine, tryptophan, and histidine.

### (Antibody That Binds to Bacterial Cells of C. Difficile or Antigen-Binding Fragment of Antibody)

The antibody of the present disclosure that binds to bacterial cells of C. difficile binds to C. difficile bacteria. The antibody of the present disclosure typically binds to unbroken C. difficile bacteria present in a culture supernatant.

As the antibody of the present disclosure that binds to bacterial cells of C. difficile, an antibody may be used that is produced from an antibody-producing cell derived from a B cell such as a hybridoma, or an antibody as a recombinant antibody may be used that is produced by introducing a nucleic acid encoding the antibody into a cell other than immune cells using a genetic recombination technique.

The antibody of the present disclosure that binds to bacterial cells of C. difficile is preferably an IgA antibody.

In one aspect, the antibody of the present disclosure that binds to bacterial cells of C. difficile comprises:
a heavy chain variable region comprising an amino acid sequence of a heavy chain CDR1, an amino acid sequence of a heavy chain CDR2, and an amino acid sequence of a heavy chain CDR3 of a heavy chain variable region comprising an amino acid sequence represented by SEQ ID NO: 3; and
a light chain variable region comprising an amino acid sequence of a light chain CDR1, an amino acid sequence of a light chain CDR2, and an amino acid sequence of a light chain CDR3 of a light chain variable region comprising an amino acid sequence represented by SEQ ID NO: 4.

| Sequence name (SEQ ID NO) | Amino acid sequence |
|---|---|
| SNK0004_HV (SEQ ID NO: 3) | MKFSWVIFFLMAVVTGVNSEVQLQQSLAELVRPGAS VKLSCTASGFNIKNTYMHWVKQRPEQDLEWIGRIDP ANGYTKYAPKFQGKATITADTSSNAAYLQLSSLTSE DTAIYYCGRGYSNFDYWGQGTTLTVSS |
| SNK0004_LV (SEQ ID NO: 4) | MDMRTPAQFLGILLLWFPGFKCDIKMTQSPSSMYAS LGERVTITCKASQDINSYLSWFQQKPGKSPKTLTYR ANRLVDGVPSKFSGSGSGQDYSLTISSLEYEDMGIY YCLQYDEFPLTFGAGTKLELK |

In one aspect, the antibody of the present disclosure that binds to bacterial cells of C. difficile comprises a heavy chain variable region comprising:
a heavy chain CDR1 comprising an amino acid sequence represented by SEQ ID NO: 5;
a heavy chain CDR2 comprising an amino acid sequence represented by SEQ ID NO: 6; and
a heavy chain CDR3 comprising an amino acid sequence represented by SEQ ID NO: 7, and comprises a light chain variable region comprising:
a light chain CDR1 comprising an amino acid sequence represented by SEQ ID NO: 8;
a light chain CDR2 comprising an amino acid sequence represented by SEQ ID NO: 9; and
a light chain CDR3 comprising an amino acid sequence represented by SEQ ID NO: 10.

| Sequence name (SEQ ID NO) | Amino acid sequence |
|---|---|
| SNK0004_HCDR1 (SEQ ID NO: 5) | GFNIKNTY |
| SNK0004_HCDR2 (SEQ ID NO: 6) | IDPANGYT |
| SNK0004_HCDR3 (SEQ ID NO: 7) | GRGYSNFDY |
| SNK0004_LCDR1 (SEQ ID NO: 8) | QDINSY |
| SNK0004_LCDR2 (SEQ ID NO: 9) | RAN |
| SNK0004_LCDR3 (SEQ ID NO: 10) | LQYDEFPLT |

In one aspect, the antibody of the present disclosure that binds to bacterial cells of C. difficile comprises:
a heavy chain variable region comprising an amino acid sequence represented by SEQ ID NO: 1 or a sequence having a sequence identity with this amino acid sequence of at least 90%, at least 95%, at least 98%, or at least 99%; and
a light chain variable region comprising an amino acid sequence represented by SEQ ID NO: 2 or a sequence having a sequence identity with this amino acid sequence of at least 90%, at least 95%, at least 98%, or at least 99%.

In one aspect, the antibody of the present disclosure that binds to bacterial cells of C. difficile comprises:
a heavy chain variable region comprising an amino acid sequence represented by SEQ ID NO: 3 or a sequence having a sequence identity with this amino acid sequence of at least 90%, at least 95%, at least 98%, or at least 99%; and
a light chain variable region comprising an amino acid sequence represented by SEQ ID NO: 4 or a sequence having a sequence identity with this amino acid sequence of at least 90%, at least 95%, at least 98%, or at least 99%, and the antibody comprises the heavy chain variable region comprising:
   a heavy chain CDR1 comprising an amino acid sequence represented by SEQ ID NO: 5;
   a heavy chain CDR2 comprising an amino acid sequence represented by SEQ ID NO: 6; and
   a heavy chain CDR3 comprising an amino acid sequence represented by SEQ ID NO: 7, and comprises the light chain variable region comprising:
      a light chain CDR1 comprising an amino acid sequence represented by SEQ ID NO: 8;
      a light chain CDR2 comprising an amino acid sequence represented by SEQ ID NO: 9; and
      a light chain CDR3 comprising an amino acid sequence represented by SEQ ID NO: 10.

In one aspect, the IgA antibody of the present disclosure is SNK0004 obtained by determining a nucleic acid sequence encoding an IgA antibody produced by a hybridoma obtained from a mouse-derived B cell and applying a recombination technique to obtain an IgA antibody.

The antibody SNK0004 has the following amino acid sequence components.

| Amino acid sequence of SNK0004 | | |
|---|---|---|
| Heavy chain | Full-length sequence | MKFSWVIFFLMAVVTGVNSEVQLQQSLAELVRPGASVKLSCTASGF NIKNTYMHWVKQRPEQDLEWIGRIDPANGYTKYAPKFQGKATITAD TSSNAAYLQLSSLTSEDTAIYYCGRGYSNFDYWGQGTTLTVSSESA RNPTIYPLTLPRALSSDPVIIGCLIHDYFPSGTMNVTWGKSGKDIT TVNFPPALASGGGYTMSSQLTLPAVECPEGESVKCSVQHDSNAVQE LDVKCSGPPPPCPPCPPSCHPSLSLQRPALEDLLLGSDASLTCTLN GLRNPEGAVFTWEPSTGKDAVQKKAVQNSCGCYSVSSVLPGCAERW NSGASFKCTVTHPESDTLTGTIAKITVNTFPPQVHLLPPPSEELAL NELVSLTCLVRAFNPKEVLVRWLHGNEELSPESYLVFEPLKEPGEG ATTYLVTSVLRVSAELWKQGDQYSCMVGHEALPMNFTQKTIDRLSG KPTNVSVSVIMSEGDGICY |
| | Variable region | MKFSWVIFFLMAVVTGVNSEVQLQQSLAELVRPGASVKLSCTASGF NIKNTYMHWVKQRPEQDLEWIGRIDPANGYTKYAPKFQGKATITAD TSSNAAYLQLSSLTSEDTAIYYCGRGYSNFDYWGQGTTLTVSS |
| | CDR1 | GFNIKNTY |
| | CDR2 | IDPANGYT |
| | CDR3 | GRGYSNFDY |
| Light chain | Full-length sequence | MDMRTPAQFLGILLLWFPGFKCDIKMTQSPSSMYASLGERVTITCK ASQDINSYLSWFQQKPGKSPKTLIYRANRLVDGVPSKFSGSGSGQD YSLTISSLEYEDMGIYYCLQYDEFPLTFGAGTKLELKRADAAPTVS IFPPSSEQLTSGGASVVCFLNNFYPKDINVKWKIDGSERQNGVLNS WTDQDSKDSTYSMSSTLTLTKDEYERHNSYTCEATHKTSTSPIVKS FNRNEC |
| | Variable region | MDMRTPAQFLGILLLWFPGFKCDIKMTQSPSSMYASLGERVTITCK ASQDINSYLSWFQQKPGKSPKTLIYRANRLVDGVPSKFSGSGSGQD YSLTISSLEYEDMGIYYCLQYDEFPLTFGAGTKLELK |
| | CDR1 | QDINSY |
| | CDR2 | RAN |
| | CDR3 | LQYDEFPLT |

In one aspect, the antibody SNK0004 is encoded by the following base sequences.

| Sequence name (SEQ ID NO) | Base sequence |
|---|---|
| Heavy chain | ATGAAATTCAGCTGGGTCATCTTCTTCCTGATGGCAGTGGTT ACAGGGGTCAATTCAGAGGTTCAGCTGCAGCAGTCTCTGGCA GAGCTTGTGAGGCCAGGGGCCTCAGTCAAGTTGTCCTGCACA GCTTCTGGCTTCAACATCAAAAACACCTATATGCACTGGGTG AAGCAGAGGCCTGAACAGGACCTGGAGTGGATTGGAAGGATT GATCCTGCGAATGGTTATACTAAATATGCCCCGAAGTTCCAG GGCAAGGCCACTATAACTGCAGACACATCCTCCAACGCAGCC TACCTGCAGCTCAGCAGCCTGACATCTGAGGACACTGCCATC TATTACTGTGGTAGAGGCTATAGTAACTTTGACTATTGGGGC CAAGGCACCACTCTCACAGTCTCCTCAGAGTCTGCGAGAAAT CCCACCATCTACCCACTGACACTCCCACGAGCTCTGTCAAGT GACCCAGTGATAATCGGCTGCCTGATTCACGATTACTTCCCT TCCGGCACGATGAATGTGACCTGGGGAAAGAGTGGGAAGGAT ATAACCACCGTAAACTTCCCACCTGCCCTGGCCTCTGGGGGA GGGTACACCATGAGCAGCCAGTTGACCCTGCCAGCTGTCGAG TGCCCAGAAGGAGAATCCGTGAAATGTTCCGTGCAACATGAC TCTAACGCCGTCCAAGAATTGGATGTGAAGTGCTCTGGTCCT CCTCCTCCTTGTCCTCCTTGTCCTCCTTCCTGCCATCCCAGC CTGTCACTGCAGCGGCCAGCTCTTGAGGACCTGCTCCTGGGT TCAGATGCCAGCCTCACATGTACTCTGAATGGCCTGAGAAAT CCTGAGGGAGCTGTCTTCACCTGGGAGCCCTCCACTGGGAAG GATGCAGTGCAGAAGAAAGCTGTGCAGAATTCCTGCGGCTGC TACAGTGTGTCCAGCGTCCTGCCTGGCTGTGCTGAGCGCTGG AACAGTGGCGCATCATTCAAGTGCACAGTTACCCATCCTGAG TCTGACACCTTAACTGGCACAATTGCCAAAATCACAGTGAAC ACCTTCCCACCCCAGGTCCACCTGCTACCGCCGCCGTCGGAG GAGCTGGCCCTGAATGAGCTCGTGTCCCTGACATGCCTGGTG CGAGCTTTCAACCCTAAAGAAGTGCTGGTGCGATGGCTGCAT GGAAATGAGGAGCTGTCCCCAGAAAGCTACCTAGTGTTTGAG CCCCTAAAGGAGCCAGGCGAGGGAGCCACCACCTACCTGGTG ACAAGCGTGTTGCGTGTATCAGCTGAACTCTGGAAACAGGGT |
| SNK0004_nHC (SEQ ID NO: 11) | |
| | GACCAGTACTCCTGCATGGTGGGCCACGAGGCCTTGCCCATG AACTTCACCCAGAAGACCATCGACCGTCTGTCGGGTAAACCC ACCAATGTCAGCGTGTCTGTGATCATGTCAGAGGGAGATGGC ATCTGCTACTGA |
| Light chain | ATGGACATGAGGACCCCTGCTCAGTTTCTTGGAATCTTGTTG CTCTGGTTTCCAGGTTTCAAATGTGACATCAAGATGACCCAG TCTCCATCTTCCATGTATGCATCTCTAGGAGAGAGAGTCACT ATCACTTGCAAGGCGAGTCAGGACATAAATAGTTATTTAAGC TGGTTCCAGCAGAAACCAGGGAAATCTCCTAAGACCCTGACC TATCGTGCAAACAGATTGGTAGATGGGGTCCCATCAAAATTC AGTGGCAGTGGATCTGGGCAAGATTATTCTCTCACCATCAGC AGCCTGGAGTATGAAGATATGGGAATTTATTATTGTCTACAG TATGATGAGTTTCCGCTCACGTTCGGTGCTGGGACCAAGCTG GAGCTGAAACGGGCTGATGCTGCACCAACTGTATCCATCTTC CCACCATCCAGTGAGCAGTTAACATCTGGAGGTGCCTCAGTC GTGTGCTTCTTGAACAACTTCTACCCCAAAGACATCAATGTC AAGTGGAAGATTGATGGCAGTGAACGACAAAATGGCGTCCTG AACAGTTGGACTGATCAGGACAGCAAAGACAGCACCTACAGC ATGAGCAGCACCCTCACGTTGACCAAGGACGAGTATGAACGA CATAACAGCTATACCTGTGAGGCCACTCACAAGACATCAACT TCACCCATTGTCAAGAGCTTCAACAGGAATGAGTGTTGA |
| SNK0004_n LC (SEQ ID NO: 12) | |

In one aspect, the antibody of the present disclosure that binds to bacterial cells of C. difficile comprises:
a heavy chain variable region comprising an amino acid sequence of a heavy chain CDR1, an amino acid sequence of a heavy chain CDR2, and an amino acid sequence of a heavy chain CDR3 of a heavy chain variable region comprising an amino acid sequence represented by SEQ ID NO: 13; and
a light chain variable region comprising an amino acid sequence of a light chain CDR1, an amino acid sequence of a light chain CDR2, and an amino acid sequence of a light chain CDR3 of a light chain variable region comprising an amino acid sequence represented by SEQ ID NO: 14.

| Sequence name (SEQ ID NO) | Amino acid sequence |
|---|---|
| **SNK0005_HV** (SEQ ID NO: 13) | MGWSWIFLLFLSGTAGVLSEVQLQQSGPEVVEPGASV KIPCKASGYTFTDYNMDWVKQSHGKSLEWIGDINPNN GGTIYNQKFKGQATLTVDKSSSTAYMELRSLTSEDTA VYYCAKSGYYGSGRYFDVWGTGTTVTVSS |
| **SNK0005_LV** (SEQ ID NO: 14) | MVSTPQFLVFLLFWIPASRGDILLTQSPVILSVSPGE RVSFSCRASQSIGSSIHWYQQRTNGSPRLLIKYASES ISGIPSRFSGSGSGTDFTLTINSVASEDIADYYCQQS KSWPWTFGGGTKLEIK |

In one aspect, the antibody of the present disclosure that binds to bacterial cells of C. difficile comprises a heavy chain variable region comprising:
a heavy chain CDR1 comprising an amino acid sequence represented by SEQ ID NO: 15;
a heavy chain CDR2 comprising an amino acid sequence represented by SEQ ID NO: 16; and
a heavy chain CDR3 comprising an amino acid sequence represented by SEQ ID NO: 17, and comprises a light chain variable region comprising:
a light chain CDR1 comprising an amino acid sequence represented by SEQ ID NO: 18;
a light chain CDR2 comprising an amino acid sequence represented by SEQ ID NO: 19; and
a light chain CDR3 comprising an amino acid sequence represented by SEQ ID NO: 20.

| Sequence name (SEQ ID NO) | Amino acid sequence |
|---|---|
| SNK0005_HCDR1 (SEQ ID NO: 15) | GYTFTDYN |
| SNK0005_HCDR2 (SEQ ID NO: 16) | INPNNGGT |
| SNK0005_HCDR3 (SEQ ID NO: 17) | AKSGYYGSGRYFDV |
| SNK0005_LCDR1 (SEQ ID NO: 18) | QSIGSS |
| SNK0005_LCDR2 (SEQ ID NO: 19) | YAS |
| SNK0005_LCDR3 (SEQ ID NO: 20) | QQSKSWPWT |

In one aspect, the antibody of the present disclosure that binds to bacterial cells of C. difficile comprises:
a heavy chain variable region comprising an amino acid sequence represented by SEQ ID NO: 13 or a sequence having a sequence identity with this amino acid sequence of at least 90%, at least 95%, at least 98%, or at least 99%; and
a light chain variable region comprising an amino acid sequence represented by SEQ ID NO: 14 or a sequence having a sequence identity with this amino acid sequence of at least 90%, at least 95%, at least 98%, or at least 99%.

In one aspect, the antibody of the present disclosure that binds to bacterial cells of C. difficile comprises:
a heavy chain variable region comprising an amino acid sequence represented by SEQ ID NO: 13 or a sequence having a sequence identity with this amino acid sequence of at least 90%, at least 95%, at least 98%, or at least 99%; and
a light chain variable region comprising an amino acid sequence represented by SEQ ID NO: 14 or a sequence having a sequence identity with this amino acid sequence of at least 90%, at least 95%, at least 98%, or at least 99%, and the antibody comprises the heavy chain variable region comprising:
   a heavy chain CDR1 comprising an amino acid sequence represented by SEQ ID NO: 15;
   a heavy chain CDR2 comprising an amino acid sequence represented by SEQ ID NO: 16; and
   a heavy chain CDR3 comprising an amino acid sequence represented by SEQ ID NO: 17, and comprises the light chain variable region comprising:
      a light chain CDR1 comprising an amino acid sequence represented by SEQ ID NO: 18;
      a light chain CDR2 comprising an amino acid sequence represented by SEQ ID NO: 19; and
      a light chain CDR3 comprising an amino acid sequence represented by SEQ ID NO: 20.

In one aspect, the IgA antibody of the present disclosure is SNK0005 obtained by determining a nucleic acid sequence encoding an IgA antibody produced by a hybridoma obtained from a mouse small intestine-derived B cell and applying a recombination technique to obtain an IgA antibody.

The antibody SNK0005 has the following amino acid sequence components.

| Amino acid sequence of SNK0005 | | |
|---|---|---|
| Heavy chain | Full-length sequence | MGWSWIFLLFLSGTAGVLSEVQLQQSGPEVVEPGASVKIPCKASGY TFTDYNMDWVKQSHGKSLEWIGDINPNNGGTIYNQKFKGQATLTVD KSSSTAYMELRSLTSEDTAVYYCAKSGYYGSGRYFDVWGTGTTVTV SSESARNPTIYPLTLPRALSSDPVIIGCLIHDYFPSGTMNVTWGKS GKDITTVNFPPALASGGGYTMSSQLTLPAVECPEGESVKCSVQHDS NAVQELDVKCSGPPPPCPPCPPSCHPSLSLQRPALEDLLLGSDASL TCTLNGLRNPEGAVFTWEPSTGKDAVQKKAVQNSCGCYSVSSVLPG CAERWNSGASFKCTVTHPESDTLTGTIAKITVNTFPPQVHLLPPPS EELALNELVSLTCLVRAFNPKEVLVRWLHGNEELSPESYLVFEPLK EPGEGATTYLVTSVLRVSAELWKQGDQYSCMVGHEALPMNFTQKTI DRLSGKPTNVSVSVIMSEGDGICY |
| | Variable region | MGWSWIFLLFLSGTAGVLSEVQLQQSGPEVVEPGASVKIPCKASGY TFTDYNMDWVKQSHGKSLEWIGDINPNNGGTIYNQKFKGQATLTVD KSSSTAYMELRSLTSEDTAVYYCAKSGYYGSGRYFDVWGTGTTVTV SS |
| | CDR1 | GYTFTDYN |
| | CDR2 | INPNNGGT |
| | CDR3 | AKSGYYGSGRYFDV |
| Light chain | Full-length sequence | MVSTPQFLVFLLFWIPASRGDILLTQSPVILSVSPGERVSFSCRAS QSIGSSIHWYQQRTNGSPRLLIKYASESISGIPSRFSGSGSGTDFT LTINSVASEDIADYYCQQSKSWPWTFGGGTKLEIKRADAAPTVSIF PPSSEQLTSGGASVVCFLNNFYPKDINVKWKIDGSERQNGVLNSWT DQDSKDSTYSMSSTLTLTKDEYERHNSYTCEATHKTSTSPIVKSFN RNEC |
| | Variable region | MVSTPQFLVFLLFWIPASRGDILLTQSPVILSVSPGERVSFSCRAS QSIGSSIHWYQQRTNGSPRLLIKYASESISGIPSRFSGSGSGTDFT LTINSVASEDIADYYCQQSKSWPWTFGGGTKLEIK |
| | CDR1 | QSIGSS |
| | CDR2 | YAS |
| | CDR3 | QQSKSWPWT |

In one aspect, the antibody SNK0005 is encoded by the following base sequences.

| Sequence name (SEQ ID NO) | Base sequence |
|---|---|
| Heavy chain | ATGGGATGGAGCTGGATCTTTCTCCTCTTCCTGTCAGGAACT GCAGGTGTCCTCTCTGAGGTCCAACTGCAACAGTCTGGACCT GAAGTGGTGGAGCCTGGGGCTTCAGTGAAGATACCCTGCAAG GCTTCTGGATACACATTCACTGACTACAACATGGACTGGGTG AAACAGAGCCATGGAAAGAGCCTTGAGTGGATTGGCGATATT AATCCGAACAATGGTGGTACTATCTACAACCAGAAGTTCAAG GGCCAGGCCACATTGACTGTTGACAAGTCCTCCAGTACAGCC TACATGGAGCTCCGCAGCCTGACATCTGAGGACACTGCAGTC TATTACTGTGCAAAATCGGGCTACTACGGTAGTGGCCGGTAC TTCGATGTCTGGGGCACAGGGACCACGGTCACCGTCTCCTCA GAGTCTGCGAGAAATCCCACCATCTACCCACTGACACTCCCA CGAGCTCTGTCAAGTGACCCAGTGATAATCGGCTGCCTGATT CACGATTACTTCCCTTCCGGCACGATGAATGTGACCTGGGGA AAGAGTGGGAAGGATATAACCACCGTAAACTTCCCACCTGCC CTGGCCTCTGGGGGAGGGTACACCATGAGCAGCCAGTTGACC CTGCCAGCTGTCGAGTGCCCAGAAGGAGAATCCGTGAAATGT TCCGTGCAACATGACTCTAACGCCGTCCAAGAATTGGATGTG AAGTGCTCTGGTCCTCCTCCTCCTTGTCCTCCTTGTCCTCCT TCCTGCCATCCCAGCCTGTCACTGCAGCGGCCAGCTCTTGAG GACCTGCTCCTGGGTTCAGATGCCAGCCTCACATGTACTCTG AATGGCCTGAGAAATCCTGAGGGAGCTGTCTTCACCTGGGAG CCCTCCACTGGGAAGGATGCAGTGCAGAAGAAAGCTGTGCAG AATTCCTGCGGCTGCTACAGTGTGTCCAGCGTCCTGCCTGGC TGTGCTGAGCGCTGGAACAGTGGCGCATCATTCAAGTGCACA GTTACCCATCCTGAGTCTGACACCTTAACTGGCACAATTGCC AAAATCACAGTGAACACCTTCCCACCCCAGGTCCACCTGCTA CCGCCGCCGTCGGAGGAGCTGGCCCTGAATGAGCTCGTGTCC CTGACATGCCTGGTGCGAGCTTTCAACCCTAAAGAAGTGCTG GTGCGATGGCTGCATGGAAATGAGGAGCTGTCCCCAGAAAGC TACCTAGTGTTTGAGCCCCTAAAGGAGCCAGGCGAGGGAGCC |
| SNK0005_nHC (SEQ ID NO: 23) | |
| | ACCACCTACCTGGTGACAAGCGTGTTGCGTGTATCAGCTGAA CTCTGGAAACAGGGTGACCAGTACTCCTGCATGGTGGGCCAC GAGGCCTTGCCCATGAACTTCACCCAGAAGACCATCGACCGT CTGTCGGGTAAACCCACCAATGTCAGCGTGTCTGTGATCATG TCAGAGGGAGATGGCATCTGCTACTGA |
| Light chain | ATGGTATCCACACCTCAGTTCCTTGTATTTTTGCTTTTCTGG ATTCCAGCCTCCAGAGGTGACATCTTGCTGACTCAGTCTCCA GTCATCCTGTCTGTGAGTCCAGGAGAAAGAGTCAGTTTCTCC TGCAGGGCCAGTCAGAGCATTGGCTCAAGCATACACTGGTAT CAGCAAAGAACAAATGGTTCTCCAAGGCTTCTCATAAAATAT GCTTCTGAGTCTATTTCTGGGATCCCTTCCAGGTTTAGTGGC AGTGGATCAGGGACAGATTTTACTCTTACCATCAACAGTGTG GCGTCTGAAGACATTGCAGATTATTACTGTCAACAAAGTAAA AGCTGGCCGTGGACGTTCGGTGGAGGCACCAAACTGGAAATC AAACGGGCTGATGCTGCACCAACTGTATCCATCTTCCCACCA TCCAGTGAGCAGTTAACATCTGGAGGTGCCTCAGTCGTGTGC TTCTTGAACAACTTCTACCCCAAAGACATCAATGTCAAGTGG AAGATTGATGGCAGTGAACGACAAAATGGCGTCCTGAACAGT TGGACTGATCAGGACAGCAAAGACAGCACCTACAGCATGAGC AGCACCCTCACGTTGACCAAGGACGAGTATGAACGACATAAC AGCTATACCTGTGAGGCCACTCACAAGACATCAACTTCACCC ATTGTCAAGAGCTTCAACAGGAATGAGTGTTGA |
| SNK0005_nLC (SEQ ID NO: 24) | |

In one aspect, the antibody according to the present disclosure can be provided in the form of an antibody comprising one heavy chain and one light chain (also referred to as a single-chain antibody) other than the form comprising two heavy chains and two light chains. The antibody according to the present disclosure can also be provided in the form of a dimer or multimer having a joining chain (J chain) and optionally having a secretary component (SC) in addition to two heavy chains and two light chains.

In one aspect, the antibody according to the present disclosure can be provided as an antibody of any class, such as IgG, IgE, IgM, IgD, IgA, or IgY, or an antibody of any subclass, such as IgG1, IgG2, IgG3, IgG4, IgA1, or IgA2.

In one aspect, the antibody according to the present disclosure may have an amino acid sequence derived from the same species or derived from different species. In the case of the amino acid sequence derived from the same species, examples of the species include a human, a mouse, a rat, a hamster, a rabbit, a goat, a donkey, a pig, a cow, a horse, a chicken, a monkey, a chimpanzee, a camel, and a llama.

The antibody having an amino acid sequence derived from different species is also not particularly limited, and examples of the antibody include antibodies derived from any two or more of a human, a mouse, a rat, a hamster, a rabbit, a goat, a sheep, a donkey, a pig, a cow, a horse, a chicken, a monkey, a chimpanzee, a camel, a llama, and the like.

### (Method of Manufacturing Antibody That Binds to Bacterial Cells of C. Difficile)

The antibody according to the present disclosure that binds to bacterial cells of C. difficile can be manufactured by performing the steps of: (1) preparing an antibody-producing immortalized cell from a B cell that is collected from intestinal mucosa lamina propria and produces the target antibody; (2) culturing the antibody-producing immortalized cell prepared in the step 1 and determining a cell that produces an antibody that binds to bacterial cells of C. difficile; and (3) recovering the antibody from the cell that produces the antibody that binds to bacterial cells of C. difficile.

In one aspect, in order to obtain a structure comprising an appropriate combination of a heavy chain variable region and/or a light chain variable region as necessary, for example, the steps may be performed that comprise a step of treating with a protease or the like, a step of introducing a functional group that enables a chemical bond such as a disulfide bond, and a step of subsequently forming the chemical bond via the functional group.

In the case of manufacturing a hybridoma by fusing a B cell and a cell that is not a B cell, the type of the cell that is not a B cell (as used herein, sometimes referred to as "another cell") is not particularly limited as long as the cell and the B cell come into contact with each other and thus fuse to form a hybridoma that does not lose the antibody-producing function exerted by the B cell described above.

Another cell as described above is preferably a cell capable of fusing with the B cell to form a hybridoma and allow the hybridoma to acquire an immortalizing function, and specific examples of another cell include cancer cells, in particular, cells derived from bone marrow species such as myeloma. Another cell is preferably myeloma, for example, a mouse NS1 cell.

The origin of another cell is not particularly limited, and examples of the origin include a human, a mouse, a rat, a hamster, a rabbit, a goat, a sheep, a donkey, a pig, a cow, a horse, a chicken, a monkey, a chimpanzee, a camel, and a llama.

The term "fusion" means that the B cell and another cell are integrally and inseparably united. Here, the phrase "integrally and inseparably" intends to exclude cell division, which is a phenomenon in which a cell after fusion grows. As described above, a fused cell is an example of a hybridoma in the present description.

Conditions at the time of mixing and fusing are not particularly limited, and conditions usually used in a method of fusing cells are to be appropriately adopted. For example, conditions used for culturing the B cell or another cell are to be appropriately modified and used. Examples of such a method include a method in which the B cell and another type of a cell that is not a B cell are mixed in an appropriate medium and brought into contact with each other in the presence of polyethylene glycol or the like, a method in which electrical stimulation is applied after the mixing, and a method in which after performing a method of using a virus such as Sendai virus, the resulting cell is incubated under the conditions of 37°C and 5% carbon dioxide.

The time for the fusion is also not particularly limited, and is to be appropriately set to the time required for completion of the fusion itself. For confirming completion of the fusion, a known method used for normal cell fusion is to be appropriately modified and performed. Examples of such a method include a method in which the degree of fusion progress is observed under a microscope, and a known kit may be appropriately selected, and cells may be fused in accordance with the conditions for use of the kit.

In one aspect, a specific recovery method is not particularly limited in the step of recovering the antibody from the cell that produces the antibody that binds to bacterial cells of C. difficile, and examples of the recovery method include a method of recovering a supernatant of a culture solution of a cell that produces an antibody, and a method of recovering a lysate of the cell. The lysate of the cell is obtained by lysing the cell by an appropriate combination of methods by known mechanical means such as ultrasonic crushing and French press and/or known chemical treatments using a surfactant, a cell wall digestive enzyme, and a cell membrane digestive enzyme, then subjecting the lysed cell to a solid-liquid separation step, and then recovering a liquid phase fraction, and thus the antibody of the present disclosure that binds to bacterial cells of C. difficile can be manufactured.

### (Method of Manufacturing Recombinant Antibody)

In one aspect, the antibody of the present disclosure that binds to bacterial cells of C. difficile is manufactured with a method in which a nucleic acid encoding the antibody is introduced into a cell capable of producing a monoclonal antibody, the cell is cultured, and a monoclonal antibody is recovered from the cell extract or the culture supernatant, and purified as necessary.

Binding of the antibody to bacterial cells of C. difficile can be confirmed by using a means well known to those skilled in the art, for example, by identifying the antibody molecule or the cell that produces the antibody using a means of ELISA, EIA, RIA, FLISA, FIA, or the like, a means of FACS, or the like.

The cell capable of producing a monoclonal antibody is usually not particularly limited as long as it is a cell capable of producing various proteins that exert functions by forming a higher order structure derived from a mammal or the like. For example, a known cell is to be appropriately selected, and examples of the cell include mammal-derived cells such as a COS cell, a HEK cell (HEK293, HEK293T, etc.), a HELA cell, and a CHO cell and insect-derived cells such as Sf9. In one aspect, a monoclonal antibody can also be produced using a yeast cell, a filamentous fungus cell, or a cell of a plant such as soybean or Arabidopsis thaliana.

A specific method of introducing a nucleic acid, a specific culture condition of a cell into which the nucleic acid is introduced, a specific recovery method, and a specific purification method depend on, for example, the type of the cell to be used, and are not particularly limited. Known methods can be appropriately modified and combined to manufacture the antibody according to the present disclosure that binds to bacterial cells of C. difficile.

In one aspect, a monoclonal antibody is produced in these cells, and then the monoclonal antibody secreted from these cells into a culture solution is dried or lyophilized together with the culture solution or the monoclonal antibody accumulated in these cells is disrupted and dried or lyophilized together with these cells, and thus the monoclonal antibody can also be manufactured as an antibody-containing composition suitable for enteral intake (Virdi et al., Nat. Biotechnol., 2019, Vol. 37, pp. 527-530). In one aspect, the cell that produces the antibody-containing composition suitable for enteral intake is preferably a yeast cell, a filamentous fungus cell, or a cell of a plant such as soybean or Arabidopsis thaliana, and the produced monoclonal antibody is preferably in a form in which a VHH fragment is fused to IgAFc.

In a case where the antibody according to the present disclosure that binds to bacterial cells of C. difficile is a chimeric antibody, the above-described nucleic acid is to be introduced into a cell capable of producing an antibody with a procedure in which nucleic acids are prepared that have base sequences encoding a heavy chain variable region and a light chain variable region and base sequences encoding a heavy chain constant region and a light chain constant region derived from different species, the produced nucleic acid having the base sequence encoding the heavy chain variable region and the nucleic acid having the base sequence encoding the heavy chain constant region are bound, the produced nucleic acid having the base sequence encoding the light chain variable region and the nucleic acid having the base sequence encoding the light chain constant region are bound, and the bound nucleic acids are introduced into a cell capable of producing an antibody as described above.

Also in a case where the antibody according to the present disclosure that binds to bacterial cells of C. difficile is an antibody in an aspect in which heavy chains CDR1 to CDR3 and/or light chains CDR1 to CDR3 have an amino acid sequence derived from a mouse and the region other than the above chains has an amino acid sequence derived from a human (sometimes referred to as a humanized antibody), the manufacturing method is to be performed, as in the case of the above-described chimeric antibody, in which nucleic acids are prepared that have base sequences encoding heavy chains CDR1 to CDR3 and/or light chains CDR1 to CDR3 and a base sequence encoding the region other than the above chains, the nucleic acids are recombined so as to constitute a heavy chain and a light chain, and these are introduced into a cell capable of producing a monoclonal IgA antibody as described above. Some bases may need to be replaced with other bases in order to maintain the binding ability of the antibody.

### (Nucleic Acid Encoding Antibody That Binds to Bacterial Cells of C. Difficile or Antigen-Binding Fragment of Antibody)

In one aspect, the present disclosure provides a nucleic acid encoding an antibody that binds to bacterial cells of C. difficile or encoding an antigen-binding fragment of the antibody. The nucleic acid encodes a heavy chain and/or a light chain of the antibody. The form of the nucleic acid is not particularly limited, and may be a ribonucleotide or a deoxynucleotide. The form of the nucleic acid is not particularly limited, and may be a single-chain form or a double-chain form. The codons used by the nucleic acid sequence are not particularly limited, and various codons can be appropriately selected and used according to the purpose. For example, various codons can be appropriately selected in consideration of codon frequency and the like according to the type of the host cell to be adopted at the time of manufacturing. In one aspect, the nucleic acid encoding the antibody of the present disclosure or encoding an antigen-binding fragment of the antibody is utilized in expressing and manufacturing the antibody according to the present disclosure or an antigen-binding fragment of the antibody.

In a case where the nucleic acid encoding the antibody of the present disclosure or encoding an antigen-binding fragment of the antibody encodes an antigen-binding fragment, for example, separate nucleic acid molecules may encode two domains of the Fv fragment, VL and VH, or a single nucleic acid may encode a single protein chain in which a pair of VL and VH regions forms a monovalent molecule (single-chain Fv (scFv)), using a recombinant technique.

In one aspect, the nucleic acid encoding the antibody of the present disclosure or encoding an antigen-binding fragment of the antibody may be in the form of a vector. For example, the vector may be an expression vector or a vector for use in genetic recombination. The components of the vector are not particularly limited, and can include various components used in a known vector.

### (Cell Including Nucleic Acid Encoding Antibody That Binds to Bacterial Cells of C. Difficile or Antigen-Binding Fragment of Antibody)

In one aspect, the present disclosure provides a cell comprising a nucleic acid encoding an antibody that binds to bacterial cells of C. difficile or encoding an antigen-binding fragment of the antibody. The cell can comprise one or more nucleic acids encoding a heavy chain and/or a light chain of the antibody of the present disclosure or an antigen-binding fragment of the antibody. In one aspect, the cell of the present disclosure produces an antibody that binds to bacterial cells of C. difficile or produces an antigen-binding fragment of the antibody. In one aspect, the cell of the present disclosure replicates a nucleic acid encoding an antibody that binds to bacterial cells of C. difficile or encoding an antigen-binding fragment of the antibody. The type of the cell of the present disclosure is not particularly limited, and cells can be used that are used for protein production, nucleic acid amplification, genetic recombination, and the like. In one aspect, as the cell of the present disclosure, the cells can be used that are exemplified in the above (Method of Manufacturing Antibody That Binds to Bacterial Cells of C. Difficile) or in the following (Antibody-Producing Immortalized Cell).

### (Antibody-Producing Immortalized Cell)

In one aspect, the present disclosure provides an immortalized cell capable of producing the above-described antibody according to the present disclosure that binds to bacterial cells of C. difficile.

A specific antibody-producing immortalized cell is a cell obtained by mixing and fusing a B cell that produces the antibody with another type of cell, that is, a cell that is not a B cell, or a cell that is infected with EB virus or the like to become an immortalized B cell.

The hybridoma may be a hybridoma derived from the same species or a hybridoma derived from different species. In the [method of manufacturing a monoclonal IgA antibody] described above, in the hybridoma derived from the same species, the B cell and the cell that is not a B cell are to be derived from the same species, and in the hybridoma derived from different species, the B cell and the cell that is not a B cell are to be derived from different species.

The origin of the hybridoma derived from the same species is to be appropriately selected, for example, from those that are an origin of a B cell and are also an origin of a cell that is not a B cell, among the origins described in the step 1 in the [method of manufacturing a monoclonal IgA antibody] described above. For example, the origin of the hybridoma derived from the same species is a human, a mouse, a rat, a hamster, a rabbit, a goat, a donkey, a pig, a cow, a horse, a chicken, a monkey, a chimpanzee, a camel, or a llama.

The origin of the hybridoma derived from different species is to be a combination of origins each appropriately selected, for example, from those that are an origin of a B cell and those that are an origin of a cell that is not a B cell, among the origins described in the step 1 in the [method of manufacturing a monoclonal IgA antibody] described above. For example, the origin of the hybridoma derived from different species is an appropriate combination of origins such as a human, a mouse, a rat, a hamster, a rabbit, a goat, a sheep, a donkey, a pig, a cow, a horse, a chicken, a monkey, and a chimpanzee.

### (Composition)

In one aspect, the present disclosure provides a composition containing the antibody according to the present disclosure that binds to bacterial cells of C. difficile, or containing an antigen-binding fragment of the antibody. The composition of the present disclosure is preferably provided as, but is not limited to, a pharmaceutical composition, an oral composition, or an enteral composition.

### (Pharmaceutical Composition)

In one aspect, the pharmaceutical composition according to the present disclosure is not particularly limited, and can be suitably used, for example, for a treatment of a disease associated with bacterial cells of C. difficile.

The disease associated with bacterial cells of C. difficile is not particularly limited, and examples of the disease include inflammatory bowel disease, ulcerative colitis, Crohn's disease, allergy, asthma, obesity, autoimmune disease, and neonatal necrotizing enterocolitis. Among them, inflammatory bowel disease is preferable.

In one aspect, the pharmaceutical composition according to the present disclosure can be suitably used for improving an intestinal bacterial flora.

Such a pharmaceutical composition according to the present disclosure is to contain the antibody according to the present disclosure that binds to bacterial cells of C. difficile or an antigen-binding fragment of the antibody in an effective amount, and for example, the content ratio of the antibody according to the present disclosure in 100 wt% of the pharmaceutical composition can be appropriately set to be in the range of 0.001 to 99.99 wt% in consideration of the type of target intestinal disease, the dosage form, the administration method, the administration subject, the degree of a symptom of the administration subject, the degree of an effect exerted by administration, and the like.

The term "effective amount" used in the present description refers to an amount such that the antibody according to the present disclosure that binds to bacterial cells of C. difficile or an antigen-binding fragment of the antibody can exert a treatment effect on intestinal disease or the like.

The pharmaceutical composition according to the present disclosure may contain a pharmaceutically acceptable carrier or additive together with the antibody according to the present disclosure that binds to bacterial cells of C. difficile. The pharmaceutically acceptable carrier or additive means any carrier, diluent, excipient, suspending agent, lubricant, adjuvant, vehicle, delivery system, emulsifier, tablet-degrading substance, absorbent, preservative, surfactant, colorant, flavor, or sweetener, and a known one is to be employed.

The pharmaceutical composition according to the present disclosure has applicability in an intestinal disease treatment method comprising a step of administering to an individual suffering from intestinal disease associated with C. difficile bacteria described above. The pharmaceutical composition also has applicability in an intestinal disease prevention method comprising a step of administering to an individual that may have a predisposition to intestinal disease without evidence of intestinal disease state or symptom as described above. These individuals are to be subjects of administration of the pharmaceutical composition according to the present disclosure.

The individual to be such a subject of administration is not particularly limited, and examples of the individual include mammals such as a human, a mouse, a rat, a guinea pig, a rabbit, a hamster, a dog, a cat, a weasel, a cow, and a pig and birds such as a chicken.

The dose and the administration method of the pharmaceutical composition are appropriately adjusted according to the type of the intestinal disease that affects the individual to be a subject of administration, the sex, the species, the age, the general condition, the seriousness of the disease, the degree of a desired effect, and the like. The dose is usually to be appropriately set in the range of 0.001 to 100 mg/kg/day.

The administration method is not particularly limited, and direct administration to the gastrointestinal tract is preferable. Examples of such an administration method include oral administration, nasal administration, transmucosal administration, and enteral administration.

The enteral administration is not limited to administration via the anus, and examples of the enteral administration include administration via a tube or the like inserted into the gastrointestinal tract from the outside of the individual, like a gastrostomy and the like. The position where a tube or the like is inserted into the gastrointestinal tract is not limited to the intestine, and examples of the position include the esophagus, the stomach, the small intestine (including the duodenum, the jejunum, the ileum, and the like), and the large intestine (including the cecum, the colon, the rectum, and the like).

In such administration of the pharmaceutical composition according to the present disclosure, the above amount of the pharmaceutical composition may be administered once a day, or may be administered in several divided doses per day. The administration interval may be every day, every other day, every week, every other week, every 2 to 3 weeks, every month, every other month, or every 2 to 3 months as long as a treatment effect on the disease is obtained.

### (Oral or Enteral Composition)

The oral or enteral composition according to the present disclosure contains the antibody according to the present disclosure that binds to bacterial cells of C. difficile, or contains an antigen-binding fragment of the antibody. Using such an oral or enteral composition enables treatment and/or prevention of a disease associated with C. difficile bacteria and improvement of an intestinal bacterial flora.

The blending ratio of the antibody that binds to bacterial cells of C. difficile in such an oral or enteral composition is not particularly limited. The blending ratio is to be appropriately adjusted according to the form, the use, and the like of the oral or enteral composition, and is usually to be about 0.001 to 99 wt% with respect to the total amount of the oral or enteral composition.

The individual to be a subject of use of the oral or enteral composition of the present disclosure is not particularly limited, and examples of the individual include mammals such as a human, a mouse, a rat, a guinea pig, a rabbit, a hamster, a dog, a cat, a weasel, a cow, and a pig and birds such as a chicken.

The antibody that binds to bacterial cells of C. difficile and is contained in the oral or enteral composition according to the present disclosure is particularly useful as an intestinal regulation composition, an intestinal environment improving composition, an intestinal environment optimizing composition, or an intestinal putrefaction preventing composition.

The amount of the oral or enteral composition according to the present disclosure used is not particularly limited as long as an effect of the oral or enteral composition as described above is exerted, and is further to be set according to the type of the individual that ingests the oral or enteral composition, the intended effect, the intended degree of the intended effect, other various conditions, and the like. Specifically, the amount of the antibody according to the present disclosure that binds to bacterial cells of C. difficile is usually to be about 0.001 to 100 mg/kg/day, and such an amount of the oral or enteral composition may be ingested in one dose to several divided doses per day.

The enteral administration is not limited to administration via the anus. For example, the enteral composition of the present disclosure can be blended with a known component and used as a bowel irrigation liquid.

The oral or enteral composition according to the present disclosure includes the antibody according to the present disclosure that binds to bacterial cells of C. difficile and exerts an effect of inhibiting abnormal growth of enteric bacteria and/or a pathological change of an intestinal bacterial flora, and can be suitably used in the field of food and the field of feed in the expectation of exerting such an effect. Thus, the oral or enteral composition of the present disclosure can be a food composition or a feed composition.

The above-described food composition is a composition obtained by suitably using the oral or enteral composition of the present disclosure exclusively in the field of food. Such a food composition can be provided as a food composition labeled as, for example, being for regulating the intestines, improving the intestinal environment, optimizing the intestinal environment, or preventing intestinal putrefaction.

Examples of the above-described food composition include general foods, and in addition, foods for specified health uses including qualified foods for specified health uses, dietary supplements, functional foods, and foods for patients.

The specific form of the above-described food composition is not particularly limited, and examples of the form include beverages such as soft drinks, carbonated beverages, nutritional beverages, fruit beverages, lactic acid beverages, and milk beverages, frozen desserts such as ice cream, ice sherbet, and shaved ice, confectioneries such as sweets, candies, gum, chocolate, tablet candies, snacks, biscuits, jelly, jam, cream, and baked confectioneries, noodles such as buckwheat noodles, Udon noodles, bean starch vermicelli, Chinese noodles, and instant noodles, processed sea foods and processed meat foods such as boiled fish pastes, ham, and sausage, dairy products such as processed milk and fermented milk, oils and fats and processed oils and fats such as salad oils, tempura oils, margarine, mayonnaise, shortening, whipped cream, and dressing, flavoring agents such as sauces and tare sauces, soups, stews, salads, side dishes, rice seasoning, pickles, bread, and cereal. In the case of foods for specified health uses, dietary supplements, functional foods, and the like, examples of the form include powders, granules, capsules, troches, tablets, and syrups.

The above-described feed composition is a composition obtained by using the feed composition of the present disclosure exclusively in the field of feed. Such a feed composition can be provided as a food composition labeled as, for example, being for regulating the intestines, improving the intestinal environment, optimizing the intestinal environment, or preventing intestinal putrefaction.

The specific form of the above-described feed composition is not particularly limited. For example, as long as an effect exerted by the feed composition according to the present disclosure described above is not impaired, the feed composition may be prepared by mixing the feed composition with a normal feed or, as necessary, with a component that can be added to a normal feed, or the feed composition itself may be used as a feed.

### (Method of Preventing or Treating Disease, or Maintaining or Improving Health)

In one aspect, the present disclosure provides a method of preventing or treating intestinal disease associated with C. difficile bacteria, or a method of maintaining or improving health associated with maintenance and improvement of an intestinal bacterial flora, and the method of preventing or treating a disease, or the method of maintaining health comprises a step of administering an effective amount of the antibody according to the present disclosure that binds to bacterial cells of C. difficile or an antigen-binding fragment of the antibody described above, the pharmaceutical composition according to the present disclosure, or the oral composition according to the present disclosure to a subject. The subject may be a subject in need of prevention or treatment of intestinal disease associated with C. difficile bacteria, or a subject in need of maintenance or improvement of health associated with maintenance and improvement of an intestinal bacterial flora.

The subject of administration described in the above [Pharmaceutical Composition] according to the present disclosure can be selected as a subject in need of prevention or treatment of intestinal disease associated with C. difficile bacteria described above. The subject in need of maintenance or improvement of health associated with maintenance and improvement of an intestinal bacterial flora is not particularly limited, and such subjects include a subject that is desired to maintain and improve the intestinal bacterial flora.

The individual to be such a subject of administration is not particularly limited, and examples of the individual include mammals such as a human, a mouse, a rat, a guinea pig, a rabbit, a hamster, a dog, a cat, a weasel, a cow, and a pig and birds such as a chicken.

The administration method and the dose to be specifically adopted can be those described above in [Pharmaceutical Composition] or [Oral or Enteral Composition] according to the present disclosure.

For example, in a case where the composition is administered as a pharmaceutical composition, the dose and the administration method of the composition are appropriately adjusted according to the type of the intestinal disease that affects the individual to be a subject of administration, the sex, the species, the age, the general condition, the seriousness of the disease, the degree of a desired effect, and the like. The dose is usually to be appropriately set in the range of 0.001 to 100 mg/kg/day.

The administration method is not particularly limited, and direct administration to the gastrointestinal tract is preferable. Examples of such an administration method include oral administration, nasal administration, transmucosal administration, and enteral administration.

The enteral administration is not limited to administration via the anus, and examples of the enteral administration include administration via a tube or the like inserted into the gastrointestinal tract from the outside of the individual, like a gastrostomy and the like. The position where a tube or the like is inserted into the gastrointestinal tract is not limited to the intestine, and examples of the position include the esophagus, the stomach, the small intestine (including the duodenum, the jejunum, the ileum, and the like), and the large intestine (including the cecum, the colon, the rectum, and the like).

In administration of the pharmaceutical composition, the above amount of the pharmaceutical composition may be administered once a day, or may be administered in several divided doses per day. The administration interval may be every day, every other day, every week, every other week, every 2 to 3 weeks, every month, every other month, or every 2 to 3 months as long as a treatment effect on the disease is obtained.

The pharmaceutical composition is to contain the antibody according to the present disclosure that binds to bacterial cells of C. difficile or an antigen-binding fragment of the antibody in an effective amount, and for example, the content ratio of the antibody according to the present disclosure in 100 wt% of the composition can be appropriately set to be in the range of 0.001 to 99.99 wt% in consideration of the type of target intestinal disease, the dosage form, the administration method, the administration subject, the degree of a symptom of the administration subject, the degree of an effect exerted by administration, and the like.

The pharmaceutical composition may contain a pharmaceutically acceptable carrier or additive together with the antibody according to the present disclosure that binds to bacterial cells of C. difficile or an antigen-binding fragment of the antibody. The pharmaceutically acceptable carrier or additive means any carrier, diluent, excipient, suspending agent, lubricant, adjuvant, vehicle, delivery system, emulsifier, tablet-degrading substance, absorbent, preservative, surfactant, colorant, flavor, or sweetener, and a known one is to be employed.

The pharmaceutical composition may contain a pharmaceutically acceptable carrier or additive together with the antibody according to the present disclosure that binds to bacterial cells of C. difficile or an antigen-binding fragment of the antibody. The pharmaceutically acceptable carrier or additive means any carrier, diluent, excipient, suspending agent, lubricant, adjuvant, vehicle, delivery system, emulsifier, tablet-degrading substance, absorbent, preservative, surfactant, colorant, flavor, or sweetener, and a known one is to be employed.

In a case where the composition is administered as the oral or enteral composition, the dose is not particularly limited as long as an effect of the composition is exerted, and is further to be set according to the type of the individual that ingests the oral or enteral composition, the intended effect, the intended degree of the intended effect, other various conditions, and the like. Specifically, the amount of the antibody according to the present disclosure that binds to bacterial cells of C. difficile is usually to be about 0.001 to 100 mg/kg/day, and such an amount of the oral or enteral composition may be ingested in one dose to several divided doses per day.

The enteral administration is not limited to administration via the anus. For example, the enteral composition of the present disclosure can be blended with a known component and used as a bowel irrigation liquid.

The oral or enteral composition can be administered as a food composition or a feed composition.

The food composition can be administered as a food composition labeled as, for example, being for regulating the intestines, improving the intestinal environment, optimizing the intestinal environment, or preventing intestinal putrefaction.

The food composition can be administered as a general food, and in addition, can be administered as a food for specified health uses, which may be a qualified food for specified health uses, a dietary supplement, a functional food, or a food for patients.

The specific form of the food composition is not particularly limited, and examples of the form include beverages such as soft drinks, carbonated beverages, nutritional beverages, fruit beverages, lactic acid beverages, and milk beverages, frozen desserts such as ice cream, ice sherbet, and shaved ice, confectioneries such as sweets, candies, gum, chocolate, tablet candies, snacks, biscuits, jelly, jam, cream, and baked confectioneries, noodles such as buckwheat noodles, Udon noodles, bean starch vermicelli, Chinese noodles, and instant noodles, processed sea foods and processed meat foods such as boiled fish pastes, ham, and sausage, dairy products such as processed milk and fermented milk, oils and fats and processed oils and fats such as salad oils, tempura oils, margarine, mayonnaise, shortening, whipped cream, and dressing, flavoring agents such as sauces and tare sauces, soups, stews, salads, side dishes, rice seasoning, pickles, bread, and cereal. In a case where the food composition is a food for specified health uses, a dietary supplement, a functional food, or the like, the food composition can be administered in the form of a powder, a granule, a capsule, a troche, a tablet, a syrup, or the like.

The feed composition can be administered as a food composition labeled as, for example, being for regulating the intestines, improving the intestinal environment, optimizing the intestinal environment, or preventing intestinal putrefaction.

The specific form of the feed composition is not particularly limited. For example, as long as an effect exerted by the feed composition according to the present disclosure described above is not impaired, the feed composition may be prepared by mixing the feed composition with a normal feed or, as necessary, with a component that can be added to a normal feed, or the feed composition itself may be administered as a feed.

### Examples

Hereinafter, the present disclosure will be described in more detail with reference to Examples, but these are merely examples and do not limit the present disclosure.

### Example 1: Acquisition of Antibody That Binds to C. Difficile Cells

An antibody that binds to C. difficile cells was obtained with the following method.

### (Materials and Methods)

### (1-1) Separation of Small Intestinal and Large Intestinal Lamina Propria Cells

A mouse was euthanized and then subjected to laparotomy to remove the entire small intestine. From the removed small intestine, the connective tissue and the Peyer's patch were removed, then the small intestine was longitudinally incised, and the contents of the small intestine were washed with PBS. Next, the washed small intestine was cut into a length of about 1 cm and put into a 100 ml beaker containing 50 ml of PBS containing 1 mM EDTA. After shaking the beaker at 37°C for 20 minutes, the small intestine fragments were collected in a strainer, and the PBS containing EDTA was discarded. The small intestine fragments were put into a 50 ml tube, 20 ml of PBS was added, the resulting mixture was vigorously shaken for 10 seconds, the small intestine fragments were then collected again in a strainer, and the PBS was discarded. This operation was repeated twice, and only small intestine epithelial cells were removed from the small intestine tissue. Next, 50 ml of a digestive enzyme solution (adjusted with 100 ml RPMI 1640, 5 ml of FCS (final concentration 5%), 50 µl of 2-mercaptoethanol (final concentration 55 µM), 0.15 g of collagenase, and 1 ml of dispase (50 U/ml) (final concentration 0.5 U/ml)) warmed to 37°C was put into a 100 ml beaker, the small intestine tissue was further finely cut and added, and the resulting mixture was shaken at 37°C for 30 minutes. Then, the 100 ml beaker was allowed to stand to precipitate the small intestine tissue, and then the supernatant was transferred to a new 50 ml tube. The digestion step using the digestive enzyme solution was repeated once for 20 minutes. The liquid digest was centrifuged at 1,500 rpm at room temperature for 5 minutes, and the supernatant was discarded. Lamina propria cells obtained as precipitates were washed once with RPMI 1640 containing 2% FCS, then suspended in 2 ml of RPMI 1640 containing 2% FCS, and stored on ice. A similar operation was performed on the supernatant subjected to the reaction in the second digestion step, the first cell suspension and the second cell suspension were combined, the combined suspension was passed through a filter to remove tissue pieces, and thus small intestinal (large intestinal) lamina propria cells were obtained.

### (1-2) Preparation and Cloning of Antibody-Producing Hybridoma

The small intestinal (large intestinal) lamina propria cell or a spleen cell was fused with an NS1 cell, which is a mouse myeloma cell, to prepare a hybridoma. The cell fusion was performed in accordance with ClonaCell-HY Hybridoma Cloning Kit (STEMCELL Technologies) with the reagent and the procedure of the kit. The obtained hybridoma was grown in a methylcellulose-containing medium in accordance with ClonaCell-HY Hybridoma Cloning Kit (STEMCELL Technologies), the resulting clones were picked up with the naked eye, and each clone was further grown in a 96-well plate. Then, a frozen cell stock was prepared from the cloned hybridoma, and at the same time, the culture supernatant was acquired. For the antibody contained in the supernatant, the isotype of the antibody was confirmed with a normal sandwich ELISA method, and then the antibody titer of the antibody contained in the supernatant was measured, and thus each isotype antibody-producing hybridoma was separated. In the ELISA, the antibody used for coating of a plate was 2 ug/ml of Anti-goat-mouse IgA (Southern Biotech), Anti-goat-mouse IgG (Southern Biotech), or Anti-goat-mouse IgM (Southern Biotech), and the antibody used for detection was 0.5 µg/ml of Alkaline Phosphatase (ALP)-conjugated anti-goat mouse IgA (Southern Biotech), Alkaline Phosphatase (ALP)-conjugated anti-goat mouse IgG (Southern Biotech), or Alkaline Phosphatase (ALP)-conjugated anti-goat mouse IgM (Southern Biotech). Used control antibodies were Mouse IgAκ (Immunology Consultants Laboratory), Purified mouse IgG1κIsotype Control (BD Pharmingen), and PE-CF594 mouse IgMκIsotype Control (BD Horizon). In the measurement, OD₄₀₅ nm was measured using TriStar² LB942 (BERTHOLD TECHNOLOGIES). The above operation was performed to acquire 500 or more hybridoma clones in total.

### (1-3) Analysis of Binding Force of Hybridoma IgA Antibody to C. Difficile Cells

C. difficile was subjected to anaerobic culture (Brain Heart Infusion media, 80% N₂, 10% H₂, 10% CO₂) at 37°C, and collected by centrifugation. The bacteria were suspended in a 0.05M Na₂CO₃ buffer, and the resulting suspension was used for coating an ELISA plate. Blocking was performed with PBS containing 1% BSA, and then each antibody culture supernatant in the 96-well plate was added and reacted at room temperature for about 1 hour. Then, the plate was washed with PBS containing 0.05% Tween 20, a secondary detection antibody (described above) corresponding to each antibody isotype was added and reacted, and then a color development reaction was performed using Alkali Phosphatase tablet (Sigma). For sufficient reaction, the ELISA plate after color development was reacted overnight at 4°C, and the OD₄₀₅ nm was measured using TriStar² LB942 (BERTHOLD TECHNOLOGIES). A clone having an OD₄₀₅ nm of 2.0 or more was regarded as an antibody that binds to C. difficile cells.

The selected clone was expanded and cultured (about 100 mL culture), and antibodies were purified from the culture solution using a Protein L column (Cytiva) in the case of IgM and IgA antibodies, and using a Protein A column (Cytiva) in the case of IgG antibodies. The antibodies were eluted using a 10 mM citrate buffer (pH 2.5), the eluate was immediately neutralized with a 1 M citrate buffer (pH 9.0), and then the neutralized eluate was concentrated with Amicon (100 kDa), then dialyzed against a dialysis membrane (100 kDa pore size), and replaced with PBS. After the dialysis, the antibody solution was recovered in a clean bench, and then the antibodies were sterilized using a syringe filter (0.22 µm) and stored at 4°C. The concentration of the antibodies was measured with a sandwich ELISA method in the same manner as described above. RNA of the clone selected by ELISA was extracted, and the full length of the antibody gene was cloned and used to prepare a recombinant antibody.

### (1-4) Cloning of Full Length of Antibody Gene of Hybridoma-Derived Antibody

RNA was extracted from a cell of each clone using IsogenII (Nippon Gene Co., Ltd.). cDNA was synthesized using the extracted RNA as a template, and RT-PCR was performed using 7 types of primers (MH1-7) for a V_{H} region of the antibody and Cα, Cµ, or Cγ region-specific primers. For a light chain, PCR was performed with a Vκ primer and a Cκ primer. Table 1 shows the base sequences of the primers. The amplified V_{H} region PCR product or Vκ region PCR product was directly sequenced to acquire the variable region gene sequence of each clone. This sequence was searched with Ig blast, further upstream sequences (including signal sequences) of the specific V_{H} or Vκ gene were acquired, a PCR primer was prepared based on the most upstream sequence, and PCR was performed together with the reverse primer at the 3' end of each C region secretory sequence to acquire full-length gene base sequences of the H chain and the L chain. On the basis of the full-length gene base sequences, the H chain, the L chain, and the J chain (the base sequence was searched from database and the full-length gene sequence was acquired by PCR using cDNA of hybridoma) were cloned into pcDNA 3.1 (+) vectors (Invitrogen).

**Table 1. Base sequence of primer for amplification of antibody gene variable region of hybridoma-derived antibody**

| Primer name | Base sequence (5'-3') |
|---|---|
| MH1 | SARGTNMAGCTGSAGTC |
| MH2 | SARGTNMAGCTGSAGSAGTCWGG |
| MH3 | CAGGTTACTCTGAAAGWTSTG |
| MH4 | GAGGTCCARCTGCAACARTC |
| MH5 | CAGGTCCAACTVCAGCARCC |
| MH6 | GAGGTGAASSTGGTGGAATC |
| MH7 | GATGTGAACTTGGAAGTGTC |
| IgAR | GATGGTGGGATTTCTCGCAGAC |
| degVκ | GGCTGCAGSTTCAGTGGCAGTGGRTCWGGRAC AACGTGAGGGTGCTGCTCATG |
| CκR | |

In the base sequences in the table, S represents G or C, R represents A or G, N represents A, C, G or T, W represents A or T, V represents G, C, or A, and M represents A or C.

An Expi CHOS cell (Thermo Fischer Scientific) was transfected with the above expression vectors in accordance with the kit protocol so that the amount ratio was H : L : J = 2 : 1 : 1. (Thermo Fischer Scientific, ExpiCHO Expression System) On days 10 to 12 after transfection, the culture supernatant was recovered, and the antibodies were purified using a Protein L column, concentrated, replaced with PBS by dialysis, and then sterilized with a filter in the same manner as described above, and used for antibody titer measurement or activity test.

### Example 2: Bacteria Binding Test

Two antibodies among the antibodies obtained in Example 1, SNK0004 antibody and SNK0005 antibody were tested for the binding force to C. difficile cells. As a test method, a method was used that is similar to the method in (1-3) "Analysis of Binding Force of Hybridoma IgA Antibody to C. Difficile Cells" in Example 1.

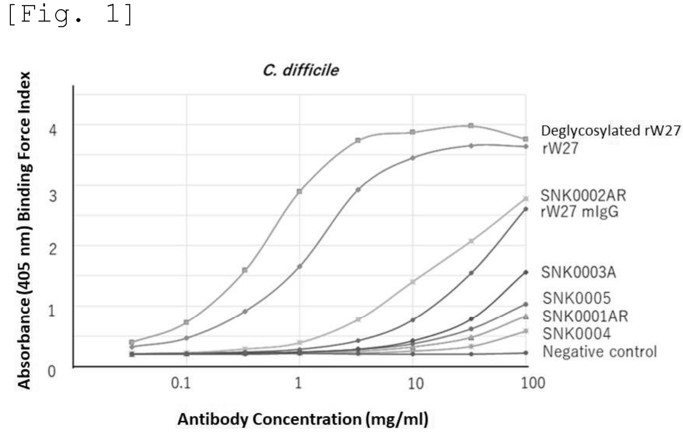

As understood from the results shown in Fig. 1, both the antibodies exhibited binding to C. difficile cells.

### Example 3: Bacterial growth inhibition test

The SNK0004 antibody, the SNK0005 antibody, and the IgG1 type antibody (rW27IgG) and the negative-control IgA antibody (Control rIgA) of W27 antibody were tested for an effect of inhibiting bacterial growth.

### (Materials and Methods)

Under the conditions similar to those in Example 1, C. difficile cells were anaerobically cultured overnight at 37°C. The culture solution was centrifuged, and the bacteria were collected and then washed twice with a culture solution. The bacterial solution was diluted 10 times with a culture solution, then Counting beads and Thiazol orange contained in Cell Viability Kit (Becton Dickinson) were added, and the number of SYTO24-positive viable bacteria was calculated by flow cytometry from the comparison with the Counting beads. The bacterial solution was diluted with a culture solution to a bacterial content of 10,000 cells/5 µl. Into an Eppendorf tube, 5 µl of the bacterial diluent was put, and 25 µl of PBS (anaerobic) or a purified test antibody (anaerobic) (antibody concentration: 1 mg/ml) was further added, and the resulting mixture was subjected to static culture at 37°C for 1 hour. Then, 30 µl of a culture solution was added, and the resulting mixture was subjected to static anaerobic culture at 37°C for 6 hours (the final concentration of the antibody was 0.42 mg/ml).

After 6 hours, the reaction solution was serially diluted, seeded on a BHI agar plate, and subjected to anaerobic culture at 37°C, and the number of viable bacteria was measured.

### (Results)

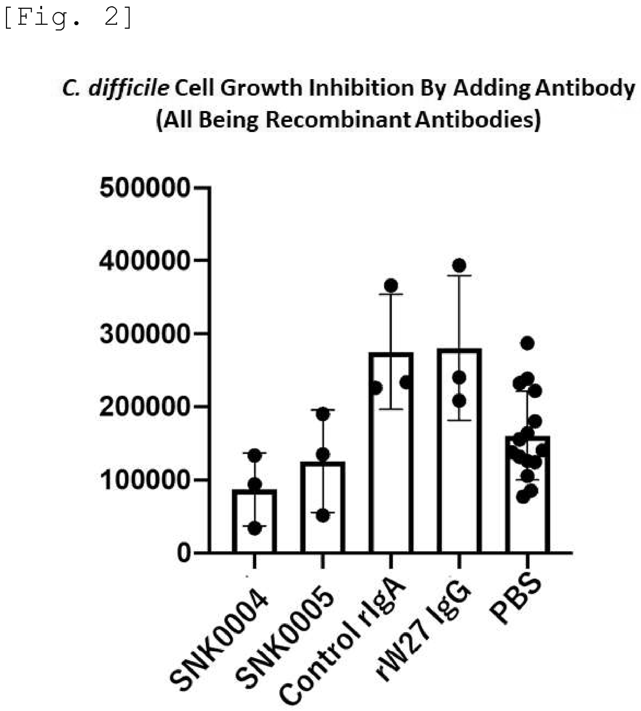

Fig. 2 shows the results. Both the SNK0004 antibody and the SNK0005 antibody tested exhibited a more remarkable C. difficile growth-inhibiting ability than the IgG1 type antibody (rW27IgG) and the negative-control IgA antibody (Control rIgA) of W27 antibody.

### Example 4: Binding property to Human Enteric Bacteria

It has been reported that abnormality of an intestinal bacterial flora (dysbiosis) is associated with the onset of many diseases including inflammatory bowel disease (IBD), and it is considered that improving an intestinal bacterial flora is important for maintaining health. The state of the intestinal bacterial flora of a patient with inflammatory bowel disease (IBD) is different from that of a healthy person, and it has been found that, for example, bacteria associated with exacerbation of IBD (IBD-related bacteria) are increased (PCT/JP2023/018019).

For IBD patient feces-derived bacteria that bind to the SNK0004 antibody and the SNK0005 antibody, the binding property of each bacterial species was evaluated with quantitative data by IgA Index (Andrew L. Kau, et al Sci Transl Med 7, 2015, Hirosuke Sugahara, et al., Frontiers in Microbiology 8, 1757, 2017).

### (Materials and Methods)

### · Analysis of Ratio of IgA Antibody-Binding Bacteria in Bacteria in Human Feces Sample

As a human feces sample, a feces of one patient (P1) was used. The operation was performed in an anaerobic chamber. The number of bacteria was measured, and then PBS was added to adjust the number of bacteria in the human feces sample to 6 × 10⁷ bacteria/ml. Then, the resulting mixture was centrifuged at 8,000 g for 5 minutes at 4°C, and the supernatant was removed. To the bacterial cells from which the supernatant was removed, 100 µl of PBS containing 20% of a normal rat serum (Wako) was added and reacted on ice for 30 minutes. Thus, non-specific binding of the antibody was inhibited. A fluorescence activated cell sorter (FACS) buffer for antibody staining was prepared by adding 10% fetal bovine serum (FBS, Nichirei Corporation) and EDTA (final concentration 5 µM, NACALAI TESQUE, INC.) to PBS and passing the resulting mixture through a 0.22 µm filter for sterilization. The FACS buffer was added to the bacterial solution, the resulting mixture was centrifuged at 8,000 g for 5 minutes at 4°C, and the supernatant was removed. With respect to 6 × 10⁶ bacteria, 15 µg of each biotinylated antibody was added and reacted on ice for 20 minutes. The FACS buffer was added to the antibody reaction solution, the resulting mixture was centrifuged at 8,000 g for 5 minutes at 4°C, and the supernatant was removed. To the bacterial cells, 20 µl of PE/Cyanine7-Streptavidin (final concentration 10 µg/ml, BioLegend) was added, and the resulting mixture was reacted on ice for 20 minutes while being shielded from light. The FACS buffer was added to the reaction solution, the resulting mixture was centrifuged at 8,000 g for 5 minutes at 4°C, and the supernatant was removed. The FACS buffer and Thiazole Orange (final concentration 42 nM, BD) were added to the bacteria and reacted on ice for 10 minutes. Then, the ratio of each antibody-binding bacteria was analyzed with SONY Cell Sorter SH800 (SONY).

### Results:

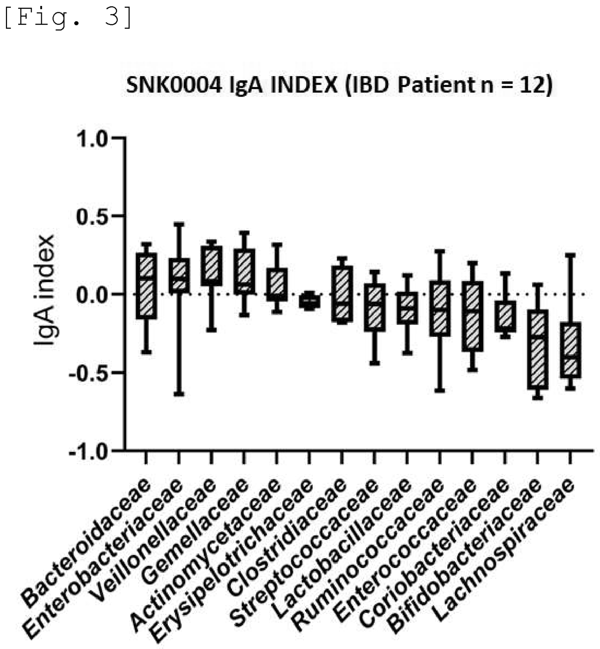

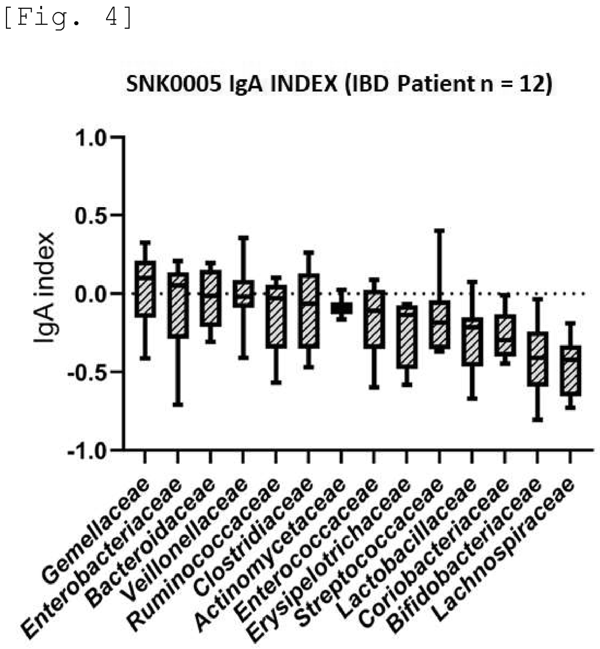

It has been found that Enterobacteriaceae exhibits specific binding to both SNK0004 and SNK0005 and other bacteria also bind to these antibodies with different binding properties (Figs. 3 and 4).

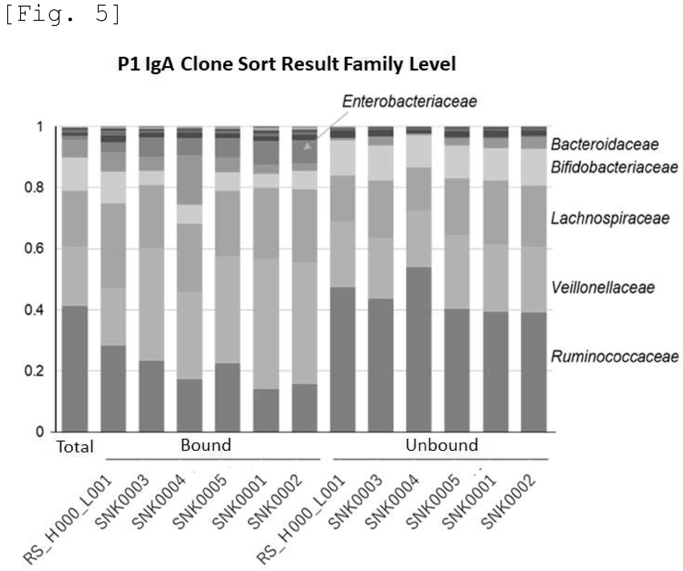

For the SNK0004 antibody, the SNK0005 antibody, and previously prepared RS_H000_L001, SNK0001, SNK0002, and SNK0003 antibodies (WO2023/277142), the ratios of IBD patient feces (feces of one patient (P1))-derived bacteria that bind to these antibodies were compared. As shown in Fig. 5, it has been considered that all of these antibodies bind to Enterobacteriaceae and have an action of improving the intestinal bacterial flora.

From the above results, it is understood that SNK0004 and SNK0005 antibodies bind to bacteria present in abnormal intestinal bacterial flora (dysbiosis) and have an action of improving the intestinal bacterial flora.

### Industrial Applicability

The present disclosure provides an antibody that specifically binds to Clostridioides difficile cells (C. difficile cells), an antigen-binding fragment of the antibody, and uses of these, and thus has an extremely high industrial utility value.

### Sequence Information

SEQ ID NO: 1: heavy chain amino acid sequence of SNK0004 
MKFSWVIFFLMAVVTGVNSEVQLQQSLAELVRPGASVKLSCTASGFNIKNTYMHWVKQRPEQDLEWI
GRIDPANGYTKYAPKFQGKATITADTSSNAAYLQLSSLTSEDTAIYYCGRGYSNFDYWGQGTTLTVS
SESARNPTIYPLTLPRALSSDPVIIGCLIHDYFPSGTMNVTWGKSGKDITTVNFPPALASGGGYTMS
SQLTLPAVECPEGESVKCSVQHDSNAVQELDVKCSGPPPPCPPCPPSCHPSLSLQRPALEDLLLGSD
ASLTCTLNGLRNPEGAVFTWEPSTGKDAVQKKAVQNSCGCYSVSSVLPGCAERWNSGASFKCTVTHP
ESDTLTGTIAKITVNTFPPQVHLLPPPSEELALNELVSLTCLVRAFNPKEVLVRWLHGNEELSPESY
LVFEPLKEPGEGATTYLVTSVLRVSAELWKQGDQYSCMVGHEALPMNFTQKTIDRLSGKPTNVSVSV
IMSEGDGICY
SEQ ID NO: 2: light chain amino acid sequence of SNK0004 
MDMRTPAQFLGILLLWFPGFKCDIKMTQSPSSMYASLGERVTITCKASQDINSYLSWFQQKPGKSPK
TLTYRANRLVDGVPSKFSGSGSGQDYSLTISSLEYEDMGIYYCLQYDEFPLTFGAGTKLELKRADAA
PTVSIFPPSSEQLTSGGASVVCFLNNFYPKDINVKWKIDGSERQNGVLNSWTDQDSKDSTYSMSSTL
TLTKDEYERHNSYTCEATHKTSTSPIVKSFNRNEC
SEQ ID NO: 3: heavy chain variable region amino acid 
sequence of SNK0004

MKFSWVIFFLMAVVTGVNSEVQLQQSLAELVRPGASVKLSCTASGFNIKNTYMHWVKQRPEQDLEWI

GRIDPANGYTKYAPKFQGKATITADTSSNAAYLQLSSLTSEDTAIYYCGRGYSNFDYWGQGTTLTVS

S
SEQ ID NO: 4: light chain variable region amino acid sequence of SNK0004 
MDMRTPAQFLGILLLWFPGFKCDIKMTQSPSSMYASLGERVTITCKASQDINSYLSWFQQKPGKSPK
TLTYRANRLVDGVPSKFSGSGSGQDYSLTISSLEYEDMGIYYCLQYDEFPLTFGAGTKLELK
SEQ ID NO: 5: heavy chain CDR1 amino acid sequence of SNK0004
   GFNIKNTY
SEQ ID NO: 6: heavy chain CDR2 amino acid sequence of SNK0004
   IDPANGYT
SEQ ID NO: 7: heavy chain CDR3 amino acid sequence of SNK0004
   GRGYSNFDY
SEQ ID NO: 8: light chain CDR1 amino acid sequence of SNK0004
   QDINSY
SEQ ID NO: 9: light chain CDR2 amino acid sequence of SNK0004
   RAN
SEQ ID NO: 10: light chain CDR3 amino acid sequence of SNK0004
   LQYDEFPLT
SEQ ID NO: 11: heavy chain base sequence of SNK0004 
ATGAAATTCAGCTGGGTCATCTTCTTCCTGATGGCAGTGGTTACAGGGGTCAATTCAGAGGTTCAGC
TGCAGCAGTCTCTGGCAGAGCTTGTGAGGCCAGGGGCCTCAGTCAAGTTGTCCTGCACAGCTTCTGG
CTTCAACATCAAAAACACCTATATGCACTGGGTGAAGCAGAGGCCTGAACAGGACCTGGAGTGGATT
GGAAGGATTGATCCTGCGAATGGTTATACTAAATATGCCCCGAAGTTCCAGGGCAAGGCCACTATAA
CTGCAGACACATCCTCCAACGCAGCCTACCTGCAGCTCAGCAGCCTGACATCTGAGGACACTGCCAT
CTATTACTGTGGTAGAGGCTATAGTAACTTTGACTATTGGGGCCAAGGCACCACTCTCACAGTCTCC
TCAGAGTCTGCGAGAAATCCCACCATCTACCCACTGACACTCCCACGAGCTCTGTCAAGTGACCCAG
TGATAATCGGCTGCCTGATTCACGATTACTTCCCTTCCGGCACGATGAATGTGACCTGGGGAAAGAG
TGGGAAGGATATAACCACCGTAAACTTCCCACCTGCCCTGGCCTCTGGGGGAGGGTACACCATGAGC
AGCCAGTTGACCCTGCCAGCTGTCGAGTGCCCAGAAGGAGAATCCGTGAAATGTTCCGTGCAACATG
ACTCTAACGCCGTCCAAGAATTGGATGTGAAGTGCTCTGGTCCTCCTCCTTGTCCTCCTTGTCC
TCCTTCCTGCCATCCCAGCCTGTCACTGCAGCGGCCAGCTCTTGAGGACCTGCTCCTGGGTTCAGAT
GCCAGCCTCACATGTACTCTGAATGGCCTGAGAAATCCTGAGGGAGCTGTCTTCACCTGGGAGCCCT
CCACTGGGAAGGATGCAGTGCAGAAGAAAGCTGTGCAGAATTCCTGCGGCTGCTACAGTGTGTCCAG
CGTCCTGCCTGGCTGTGCTGAGCGCTGGAACAGTGGCGCATCATTCAAGTGCACAGTTACCCATCCT
GAGTCTGACACCTTAACTGGCACAATTGCCAAAATCACAGTGAACACCTTCCCACCCCAGGTCCACC
TGCTACCGCCGCCGTCGGAGGAGCTGGCCCTGAATGAGCTCGTGTCCCTGACATGCCTGGTGCGAGC
TTTCAACCCTAAAGAAGTGCTGGTGCGATGGCTGCATGGAAATGAGGAGCTGTCCCCAGAAAGCTAC
CTAGTGTTTGAGCCCCTAAAGGAGCCAGGCGAGGGAGCCACCACCTACCTGGTGACAAGCGTGTTGC
GTGTATCAGCTGAACTCTGGAAACAGGGTGACCAGTACTCCTGCATGGTGGGCCACGAGGCCTTGCC
CATGAACTTCACCCAGAAGACCATCGACCGTCTGTCGGGTAAACCCACCAATGTCAGCGTGTCTGTG
ATCATGTCAGAGGGAGATGGCATCTGCTACTGA
SEQ ID NO: 12: light chain base sequence of SNK0004 
ATGGACATGAGGACCCCTGCTCAGTTTCTTGGAATCTTGTTGCTCTGGTTTCCAGGTTTCAAATGTG
ACATCAAGATGACCCAGTCTCCATCTTCCATGTATGCATCTCTAGGAGAGAGAGTCACTATCACTTG
CAAGGCGAGTCAGGACATAAATAGTTATTTAAGCTGGTTCCAGCAGAAACCAGGGAAATCTCCTAAG
ACCCTGACCTATCGTGCAAACAGATTGGTAGATGGGGTCCCATCAAAATTCAGTGGCAGTGGATCTG
GGCAAGATTATTCTCTCACCATCAGCAGCCTGGAGTATGAAGATATGGGAATTTATTATTGTCTACA
GTATGATGAGTTTCCGCTCACGTTCGGTGCTGGGACCAAGCTGGAGCTGAAACGGGCTGATGCTGCA
CCAACTGTATCCATCTTCCCACCATCCAGTGAGCAGTTAACATCTGGAGGTGCCTCAGTCGTGTGCT
TCTTGAACAACTTCTACCCCAAAGACATCAATGTCAAGTGGAAGATTGATGGCAGTGAACGACAAAA
TGGCGTCCTGAACAGTTGGACTGATCAGGACAGCAAAGACAGCACCTACAGCATGAGCAGCACCCTC
ACGTTGACCAAGGACGAGTATGAACGACATAACAGCTATACCTGTGAGGCCACTCACAAGACATCAA
CTTCACCCATTGTCAAGAGCTTCAACAGGAATGAGTGTTGA
SEQ ID NO: 13: heavy chain variable region amino acid sequence of SNK0005 
MGWSWIFLLFLSGTAGVLSEVQLQQSGPEVVEPGASVKIPCKASGYTFTDYNMDWVKQSHGKSLEWI
GDINPNNGGTIYNQKFKGQATLTVDKSSSTAYMELRSLTSEDTAVYYCAKSGYYGSGRYFDVWGTGT
TVTVSS
SEQ ID NO: 14: light chain variable region amino acid sequence of SNK0005 
MVSTPQFLVFLLFWIPASRGDILLTQSPVILSVSPGERVSFSCRASQSIGSSIHWYQQRTNGSPRLL
IKYASESISGIPSRFSGSGSGTDFTLTINSVASEDIADYYCQQSKSWPWTFGGGTKLEIK
SEQ ID NO: 15: heavy chain CDR1 amino acid sequence of SNK0005
   GYTFTDYN
SEQ ID NO: 16: heavy chain CDR2 amino acid sequence of SNK0005
   INPNNGGT
SEQ ID NO: 17: heavy chain CDR3 amino acid sequence of SNK0005
   AKSGYYGSGRYFDV
SEQ ID NO: 18: light chain CDR1 amino acid sequence of SNK0005
   QSIGSS
SEQ ID NO: 19: light chain CDR2 amino acid sequence of SNK0005
   YAS
SEQ ID NO: 20: light chain CDR3 amino acid sequence of SNK0005
   QQSKSWPWT
SEQ ID NO: 21: heavy chain amino acid sequence of SNK0005 
MGWSWIFLLFLSGTAGVLSEVQLQQSGPEVVEPGASVKIPCKASGYTFTDYNMDWVKQSHGKSLEWI
GDINPNNGGTIYNQKFKGQATLTVDKSSSTAYMELRSLTSEDTAVYYCAKSGYYGSGRYFDVWGTGT
TVTVSSESARNPTIYPLTLPRALSSDPVIIGCLIHDYFPSGTMNVTWGKSGKDITTVNFPPALASGG
GYTMSSQLTLPAVECPEGESVKCSVQHDSNAVQELDVKCSGPPPPCPPCPPSCHPSLSLQRPALEDL
LLGSDASLTCTLNGLRNPEGAVFTWEPSTGKDAVQKKAVQNSCGCYSVSSVLPGCAERWNSGASFKC
TVTHPESDTLTGTIAKITVNTFPPQVHLLPPPSEELALNELVSLTCLVRAFNPKEVLVRWLHGNEEL
SPESYLVFEPLKEPGEGATTYLVTSVLRVSAELWKQGDQYSCMVGHEALPMNFTQKTIDRLSGKPTN
VSVSVIMSEGDGICY
SEQ ID NO: 22: light chain amino acid sequence of SNK0005 
MVSTPQFLVFLLFWIPASRGDILLTQSPVILSVSPGERVSFSCRASQSIGSSIHWYQQRTNGSPRLL
IKYASESISGIPSRFSGSGSGTDFTLTINSVASEDIADYYCQQSKSWPWTFGGGTKLEIKRADAAPT
VSIFPPSSEQLTSGGASVVCFLNNFYPKDINVKWKIDGSERQNGVLNSWTDQDSKDSTYSMSSTLTL
TKDEYERHNSYTCEATHKTSTSPIVKSFNRNEC
SEQ ID NO: 23: heavy chain base sequence of SNK0005 
ATGGGATGGAGCTGGATCTTTCTCCTCTTCCTGTCAGGAACTGCAGGTGTCCTCTCTGAGGTCCAAC
TGCAACAGTCTGGACCTGAAGTGGTGGAGCCTGGGGCTTCAGTGAAGATACCCTGCAAGGCTTCTGG
ATACACATTCACTGACTACAACATGGACTGGGTGAAACAGAGCCATGGAAAGAGCCTTGAGTGGATT
GGCGATATTAATCCGAACAATGGTGGTACTATCTACAACCAGAAGTTCAAGGGCCAGGCCACATTGA
CTGTTGACAAGTCCTCCAGTACAGCCTACATGGAGCTCCGCAGCCTGACATCTGAGGACACTGCAGT
CTATTACTGTGCAAAATCGGGCTACTACGGTAGTGGCCGGTACTTCGATGTCTGGGGCACAGGGACC
ACGGTCACCGTCTCCTCAGAGTCTGCGAGAAATCCCACCATCTACCCACTGACACTCCCACGAGCTC
TGTCAAGTGACCCAGTGATAATCGGCTGCCTGATTCACGATTACTTCCCTTCCGGCACGATGAATGT
GACCTGGGGAAAGAGTGGGAAGGATATAACCACCGTAAACTTCCCACCTGCCCTGGCCTCTGGGGGA
GGGTACACCATGAGCAGCCAGTTGACCCTGCCAGCTGTCGAGTGCCCAGAAGGAGAATCCGTGAAAT
GTTCCGTGCAACATGACTCTAACGCCGTCCAAGAATTGGATGTGAAGTGCTCTGGTCCTCCTCCTCC
TTGTCCTCCTTGTCCTCCTTCCTGCCATCCCAGCCTGTCACTGCAGCGGCCAGCTCTTGAGGACCTG
CTCCTGGGTTCAGATGCCAGCCTCACATGTACTCTGAATGGCCTGAGAAATCCTGAGGGAGCTGTCT
TCACCTGGGAGCCCTCCACTGGGAAGGATGCAGTGCAGAAGAAAGCTGTGCAGAATTCCTGCGGCTG
CTACAGTGTGTCCAGCGTCCTGCCTGGCTGTGCTGAGCGCTGGAACAGTGGCGCATCATTCAAGTGC
ACAGTTACCCATCCTGAGTCTGACACCTTAACTGGCACAATTGCCAAAATCACAGTGAACACCTTCC
CACCCCAGGTCCACCTGCTACCGCCGCCGTCGGAGGAGCTGGCCCTGAATGAGCTCGTGTCCCTGAC
ATGCCTGGTGCGAGCTTTCAACCCTAAAGAAGTGCTGGTGCGATGGCTGCATGGAAATGAGGAGCTG
TCCCCAGAAAGCTACCTAGTGTTTGAGCCCCTAAAGGAGCCAGGCGAGGGAGCCACCACCTACCTGG
TGACAAGCGTGTTGCGTGTATCAGCTGAACTCTGGAAACAGGGTGACCAGTACTCCTGCATGGTGGG
CCACGAGGCCTTGCCCATGAACTTCACCCAGAAGACCATCGACCGTCTGTCGGGTAAACCCACCAAT
GTCAGCGTGTCTGTGATCATGTCAGAGGGAGATGGCATCTGCTACTGA
SEQ ID NO: 24: light chain base sequence of SNK0005

ATGGTATCCACACCTCAGTTCCTTGTATTTTTGCTTTTCTGGATTCCAGCCTCCAGAGGTGACATCT
TGCTGACTCAGTCTCCAGTCATCCTGTCTGTGAGTCCAGGAGAAAGAGTCAGTTTCTCCTGCAGGGC
CAGTCAGAGCATTGGCTCAAGCATACACTGGTATCAGCAAAGAACAAATGGTTCTCCAAGGCTTCTC
ATAAAATATGCTTCTGAGTCTATTTCTGGGATCCCTTCCAGGTTTAGTGGCAGTGGATCAGGGACAG
ATTTTACTCTTACCATCAACAGTGTGGCGTCTGAAGACATTGCAGATTATTACTGTCAACAAAGTAA
AAGCTGGCCGTGGACGTTCGGTGGAGGCACCAAACTGGAAATCAAACGGGCTGATGCTGCACCAACT
GTATCCATCTTCCCACCATCCAGTGAGCAGTTAACATCTGGAGGTGCCTCAGTCGTGTGCTTCTTGA
ACAACTTCTACCCCAAAGACATCAATGTCAAGTGGAAGATTGATGGCAGTGAACGACAAAATGGCGT
CCTGAACAGTTGGACTGATCAGGACAGCAAAGACAGCACCTACAGCATGAGCAGCACCCTCACGTTG
ACCAAGGACGAGTATGAACGACATAACAGCTATACCTGTGAGGCCACTCACAAGACATCAACTTCAC
CCATTGTCAAGAGCTTCAACAGGAATGAGTGTTGA

## Claims

1. An antibody or an antigen-binding fragment of the antibody, comprising:
a heavy chain CDR1, a heavy chain CDR2, and a heavy chain CDR3 of a heavy chain variable region represented by SEQ ID NO: 3; and
a light chain CDR1, a light chain CDR2, and a light chain CDR3 of a light chain variable region represented by SEQ ID NO: 4, or comprising:
a heavy chain CDR1, a heavy chain CDR2, and a heavy chain CDR3 of a heavy chain variable region represented by SEQ ID NO: 13; and
a light chain CDR1, a light chain CDR2, and a light chain CDR3 of a light chain variable region represented by SEQ ID NO: 14,
the antibody or the antigen-binding fragment that binds to bacterial cells of Clostridioides difficile (C. difficile) and inhibits growth of Clostridioides difficile.

2. The antibody or the antigen-binding fragment according to claim 1, wherein
the heavy chain CDR1 is represented by SEQ ID NO: 5, the heavy chain CDR2 is represented by SEQ ID NO: 6, and the heavy chain CDR3 is represented by SEQ ID NO: 7, and
the light chain CDR1 is represented by SEQ ID NO: 8, the light chain CDR2 is represented by SEQ ID NO: 9, and the light chain CDR3 is represented by SEQ ID NO: 10.

3. The antibody or the antigen-binding fragment according to claim 1, wherein
the heavy chain CDR1 is represented by SEQ ID NO: 15, the heavy chain CDR2 is represented by SEQ ID NO: 16, and the heavy chain CDR3 is represented by SEQ ID NO: 17, and
the light chain CDR1 is represented by SEQ ID NO: 18, the light chain CDR2 is represented by SEQ ID NO: 19, and the light chain CDR3 is represented by SEQ ID NO: 20.

4. A nucleic acid encoding the antibody or the antigen-binding fragment according to claim 1.

5. An expression vector comprising the nucleic acid according to claim 4.

6. A cell comprising the nucleic acid according to claim 4.

7. A composition comprising the antibody or the antigen-binding fragment according to claim 1.

8. The composition according to claim 7, being in a lyophilized state.
